(19) Europäisches Patentamt
European Patent Office
Office européen des brevets

(11) EP 0 662 952 B1

(12) **EUROPÄISCHE PATENTSCHRIFT**

(45) Veröffentlichungstag und Bekanntmachung des Hinweises auf die Patenterteilung:
**25.08.1999 Patentblatt 1999/34**

(21) Anmeldenummer: **93921894.7**

(22) Anmeldetag: **30.09.1993**

(51) Int Cl.⁶: **C07C 315/00**, C07C 317/44, C07C 323/52, C07C 323/56

(86) Internationale Anmeldenummer:
**PCT/EP93/02674**

(87) Internationale Veröffentlichungsnummer:
**WO 94/07850 (14.04.1994 Gazette 1994/09)**

(54) **VERFAHREN ZUR STEREOSELEKTIVEN SYNTHESE VON 3-SUBSTITUIERTEN 2-SULFONYLMETHYLPROPIONSÄUREN SOWIE ZWISCHENPRODUKTE**

PROCESS FOR THE STEREOSELECTIVE SYNTHESIS OF 3-SUBSTITUTED 2-SULPHONYL METHYL PROPIONIC ACIDS AND INTERMEDIATE PRODUCTS

PROCEDE DE SYNTHESE STEREOSELECTIVE D'ACIDES 2-SULFONYLMETHYLPROPIONIQUES SUBSTITUES EN POSITION 3, AINSI QUE LEURS PRODUITS INTERMEDIAIRES

(84) Benannte Vertragsstaaten:
**AT BE CH DE DK ES FR GB GR IE IT LI LU NL PT SE**

(30) Priorität: **01.10.1992 DE 4233100**

(43) Veröffentlichungstag der Anmeldung:
**19.07.1995 Patentblatt 1995/29**

(73) Patentinhaber: **HOECHST AKTIENGESELLSCHAFT**
**65929 Frankfurt am Main (DE)**

(72) Erfinder:
- **BECK, Gerhard**
  **D-60320 Frankfurt am Main (DE)**
- **JENDRALLA, Joachim-Heiner**
  **D-65931 Frankfurt am Main (DE)**
- **KAMMERMEIER, Bernhard**
  **D-60529 Frankfurt am Main (DE)**

(56) Entgegenhaltungen:
**EP-A- 0 309 766** **WO-A-92/03132**
**US-A- 4 758 584**

- **JOURNAL OF MEDICINAL CHEMISTRY, Bd. 31, Nr. 9 , September 1988 Washington, DC, US, Seiten 1839 - 1846 P. BÜHLMAYER, ET AL.: 'Synthesis and biological activity if some transition-state inhibitors of human renin' in der Anmeldung erwähnt**
- **JOURNAL OF ORGANIC CHEMISTRY, Bd. 43, Nr. 6 , 17. März 1978 Washington, DC, US, Seiten 1259 - 1262 M.F. SEMMELHACK, ET AL.: 'Preparation of 2-(alkylthiomethyl)acrylates'**
- **TETRAHEDRON LETTERS, Bd. 33, Nr. 33 , 11. August 1992 Oxford, GB, Seiten 4713 - 4716 J.H. HUTCHINSON, ET AL.: 'Formation of a novel thiopyranoindole ring system'**
- **SYNTHETIC COMMUNICATIONS, Bd. 21, Nr. 4 , 1991 New York, US, Seiten 1481 - 1487 D. COLOMBARNI, ET AL.: 'Synthesis of (3-ethoxycarbonyl but-3-en-2-yl)phenyl sulphone and (2-ethoxycarbonyl but-3-en-2-yl)phenyl sulphone from ethyl 2-bromomethyl but-2-enoate'**
- **SOUTH AFRICAN JOURNAL OF CHEMISTRY, Bd. 40, Nr. 1 , März 1987 Pretoria, ZA, Seiten 35 - 38 F. AMEER, ET AL.: 'Necic acid synthons. Part 7. 2-Substituted acrylate systems. Useful extensions to the 1,4-diazabicyclo[2.2.2]octane-derived cascade'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

3-Substituierte 2-Sulfonylmethylpropionsäuren und deren Derivate, z. B. Ester verdienen Interesse als Bausteine sowie als Vorstufen für Aspartylprotease-Inhibitoren [P. Bühlmeyer et al., J. Med. Chem. 31 (1988), 1839; R. Henning, Nachr. Chem. Techn. Lab. 38 (1990), 460; J. R. Huff, J. Med. Chem. 34 (1991), 2305].

Die genannten Verbindungen sind bereits bekannt. Es ist weiterhin eine Reihe von Synthesen bzw. Verfahren zur Herstellung dieser Verbindungen beschrieben.

Stellvertretend seien hier einige aktuelle Arbeiten genannt:

P. Bühlmeyer et al., J. Med. Chem. 31 (1988), 1839; K. Tsuji et al., Tetrahedron Lett. 30 (1989), 6189; M. Nakano et al., Tetrahedron Lett. 31 (1990), 1569; M. Nakano et al., Chem. Lett. (1990), 505; vgl. auch D. A. Evans et al., J. Org. Chem. 50 (1985), 1830.

Die erstgenannte Synthese erfordert unter anderem die Verwendung von Formaldehyd bzw. Formaldehyd-Derivaten, die ebenso wie dabei entstehende Nebenprodukte aufgrund ihrer alkylierenden Eigenschaften als gesundheitlich äußerst bedenklich gelten und spezielle, auch arbeitshygienische Sicherheitsvorkehrungen [Merck-Index 11, 4150] erfordern.

Darüber hinaus ist zur Herstellung optisch reiner Verbindungen eine Racematspaltung erforderlich. Dazu wird die racemische Säure mit L-Phenylalaninol in die diastereomeren Amide überführt, diese chromatographisch getrennt und das gewünschte Isomere durch Hydrolyse des entsprechenden Amids erhalten. Die Vielzahl der Stufen, die grundsätzlichen Nachteile einer Racematspaltung und die Probleme, die eine chromatographische Reinigung beim scale up verursacht, lassen diese Route für die Herstellung größerer Mengen nicht attraktiv erscheinen.

Die Synthese nach Tsuyi et al. führt bei ähnlicher Stufenzahl, jedoch ohne Racematspaltung, zu optisch reinem Material. Diese Synthese umfaßt eine Reihe von Schritten zur Einführung und Abspaltung von Schutzgruppen; es werden zum Teil teure Reagenzen eingesetzt, karzinogene Formaldehyd-Derivate verwendet bzw. im Laufe der Synthesen hergestellt [H. G. Neumann in "Allgemeine und spezielle Pharmakologie und Toxikologie", 4. Auflage, W. Forth, Hrsg., B. I. Wissenschaftsverlag, Mannheim-Wien-Zürich, S. 621 ff (1983); Arch. Environ. Health 30 (2), 61]. Für die Darstellung großer Mengen stellt dieser Syntheseweg somit keine ökonomisch und ökologisch vertretbare Alternative dar.

Die im Rahmen der von Evans und Mathre beschriebenen Thiorphan-Synthese geschilderte Darstellung von 2-Mercaptomethyldihydrozimtsäure ist im Vergleich zu den beiden bereits diskutierten Synthesen kurz, verläuft mit guter Gesamtausbeute und ermöglicht die gezielte Darstellung beider Enantiomerer in hoher optischer Reinheit. Schwachpunkt dieser Synthese ist die umständliche Einführung der Mercaptangruppierung und die dazu nötige Verwendung des gesundheitlich bedenklichen Benzylthiomethylbromids, welches aus Trioxan (als Formaldehyd-Quelle), Benzylmercaptan (bzw. Benzylthiomethylchlorid) und HBr dargestellt wird [H. G. Neumann in "Allgemeine und spezielle Pharmakologie und Toxikologie", 4. Auflage, W. Forth, Hrsg., B. I. Wissenschaftsverlag, Mannheim-Wien-Zürich, S. 621 ff (1983); Arch. Environ. Health 30 (2), 61].

Nakano et al. beschreiben zwei Routen zum Aufbau des Kohlenstoffgerüstes der Verbindungen I. Die erste Synthese geht aus von Malonsäurediethylester, erfordert sechs Stufen und zusätzlich eine chromatographische Diastereomeren-Trennung.

Die zweite Route verläuft über ein chirales, nicht-racemisches Arylpropionyloxazolidinon und dessen stereoselektiver Alkylierung mit Benzylbrommethylether [M. W. Holladay et al., J. Med. Chem. 30 (1987), 374] und anschließender Chromatographie. Der Schwefelsubstituent wird nach Entfemung der Benzylgruppe durch Hydrierung - wie bei der ersten Route - durch Tosylierung der freien Hydroxygruppe und anschließender Substitution mit $NaSCH_2CH_3$ in DMF eingeführt.

Auch diese beiden Synthesewege sind gekennzeichnet durch: hohe Stufenzahl, karzinogene bzw. toxische Formaldehyd-Derivate als Vorstufen bzw. Reagenzien, die umständliche und nicht ganz racemisierungsfreie Einführung des Schwefelsubstituenten und nicht zuletzt die chromatographische Abtrennung von Verunreinigungen und diastereomeren Verbindungen.

Der vorliegenden Erfindung liegt die Aufgabe zugrunde ein Verfahren zur Synthese von Verbindungen der Formel I zu entwickeln, das

* eine geringe Stufenzahl aufweist,
* keine Schutzgruppenchemie erfordert,
* keine säulenchromatographischen Schritte erfordert,
* stereoselektiv ist und wahlweise zu den enantiomeren Verbindungen der Formel I führt,
* keine Racematspaltung oder Diastereomerentrennung erfordert und
* unter gesundheitlichen, ökologischen und sicherheitstechnischen Gesichtspunkten eine Verbesserung darstellt bzw. unbedenklich ist.

Diese Aufgabe wird durch das erfindungsgemäße Verfahren gelöst. Erfindungsgegenstand ist demzufolge ein Verfahren zur stereoselektiven Herstellung einer Verbindung der Formel I

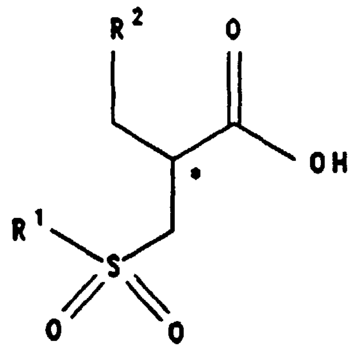

I

worin

$R^1$ $C_1$-$C_6$-Alkyl, $C_3$-$C_6$-Cycloalkyl, ($C_6$-$C_{12}$)-Aryl-($C_1$-$C_4$)-alkyl oder $C_6$-$C_{12}$-Aryl bzw. -Heteroaryl bzw. -Heterocycloalkyl, die mit 1, 2 oder 3 gleichen oder verschiedenen Hydroxy-, Methoxy- oder Trialkylsilyloxygruppen substituiert sein können; und

$R^2$ $C_6$-$C_{12}$-Aryl, das mit 1, 2 oder 3 gleichen oder verschiedenen Methoxy-, Halogen-, Cyano-, Methyl-, Trifluormethyl-, Isopropyl- oder Nitrogruppen substituiert sein kann;

$C_3$-$C_9$-Heteroaryl, das mit 1, 2 oder 3 gleichen oder verschiedenen Methoxy-, Halogen-, Cyano-, Methyl-, Trifluormethyl-, Isopropyl- oder Nitrogruppen substituiert sein kann; oder

$C_1$-$C_{10}$-Alkyl, Alkenyl oder Alkinyl, die durch 1, 2 oder 3 gleiche oder verschiedene Methoxy-, Halogen-, Cyano-, Methyl-, Trifluormethyl-, Isopropyl- oder Nitrogruppen substituiert sein können, bedeuten

und die Verbindungen der Formel I in der R- oder S-Form vorliegen können, dadurch gekennzeichnet, daß man

I) Mercaptane der Formel II

$$R^1\text{-SH} \qquad \text{II}$$

wobei $R^1$ die zur Formel I angegebene Bedeutung hat, in Gegenwart einer Base umsetzt mit einer Verbindung der Formel III

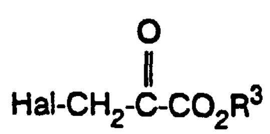

III

wobei Hal = Jod, Brom oder Chlor sein kann und $R^3$ ein geradkettiger oder verzweigter Alkylrest mit 1 bis 6 C-Atomen sein kann, zu einer Verbindung der Formel IV

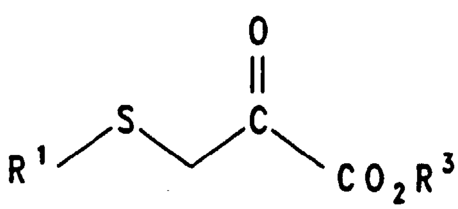

IV

wobei $R^1$ die zu Formel I und $R^3$ die zu Formel III angegebene Bedeutung haben, und

II) die Verbindungen der Formel IV mit Yliden der Formel V

$$R^2\text{-}CH{=}P(C_6H_5)_3 \qquad V$$

wobei $R^2$ die zur Formel I gegebene Bedeutung hat, umsetzt zu den ungesättigten Verbindungen der Formel VI

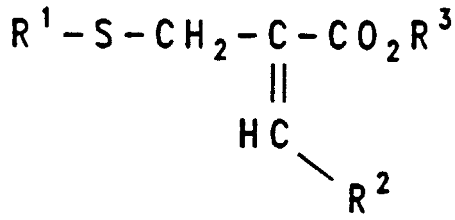

VI

wobei $R^1$ und $R^2$ die zu Formel I und $R^3$ die zu Formel III angegebene Bedeutung haben, und

III) die Verbindungen der Formel VI überführt in Verbindungen der Formel I indem man

A) die Verbindungen der Formel VI alkalisch verseift zu Verbindungen der Formel VII,

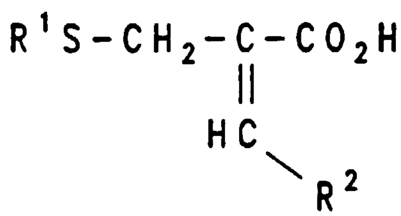

VII

worin $R^1$ und $R^2$ die zur Formel I angegebene Bedeutung haben, und durch

a) enantioselektive Hydrierung Verbindungen der Formel VII in Verbindungen der Formel X überführt

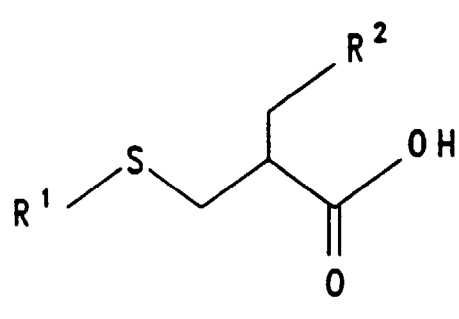

X

worin $R^1$ und $R^2$ die zur Formel I angegebene Bedeutung haben, und durch anschließende Oxidation Verbindungen der Formel X in Verbindungen der Formel I überführt
oder
b) durch Oxidation Verbindungen der Formel VII zu Verbindungen der Formel XI umsetzt

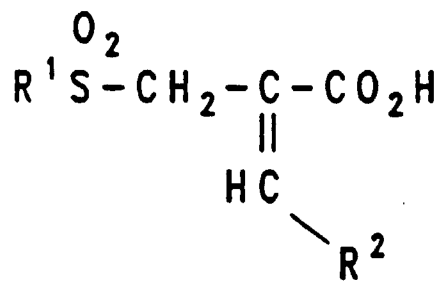

XI

worin $R^1$ und $R^2$ die zur Formel I angegebene Bedeutung haben, und durch anschließende enantioselektive Hydrierung der Verbindungen der Formel XI Verbindungen der Formel I erhält,
oder

B) die Verbindungen der Formel VI mit chiralen Katalysatoren enantioselektiv hydriert zu den Verbindungen der Formel VIII

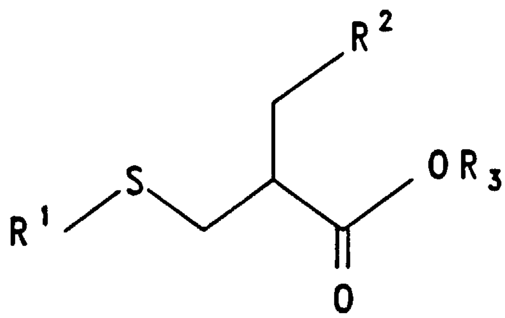

VIII

worin $R^1$ und $R^2$ die zur Formel I und $R^3$ die zur Formel III angegebene Bedeutung haben,
und durch

a) alkalische Verseifung Verbindungen der Formel VIII in Verbindungen der Formel X

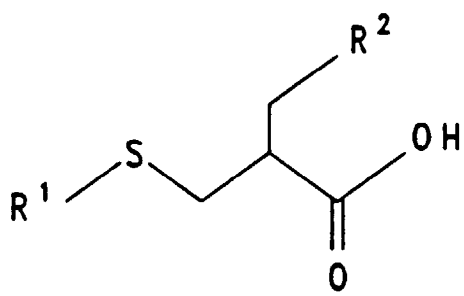

X

worin $R^1$ und $R^2$ die zur Formel I angegebene Bedeutung haben, überführt und durch anschließende Oxidation der Verbindungen der Formel X Verbindungen der Formel I erhält,
oder
b) durch Oxidation der Verbindungen der Formel VIII Verbindungen der Formel XII erhält,

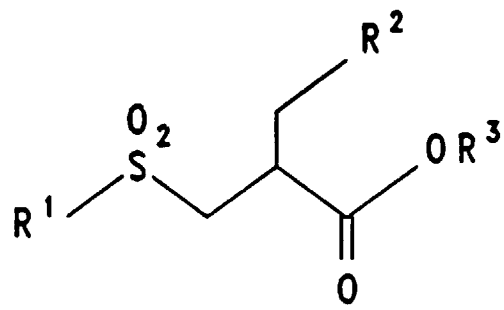

XII

worin $R^1$ und $R^2$ die zur Formel I und $R^3$ die zur Formel III angegebene Bedeutung haben und anschließend durch alkalische Verseifung Verbindungen der Formel XII in Verbindungen der Formel I überführt, oder

C) die Verbindungen der Formel VI oxydiert zu den Verbindungen der Formel IX, wobei

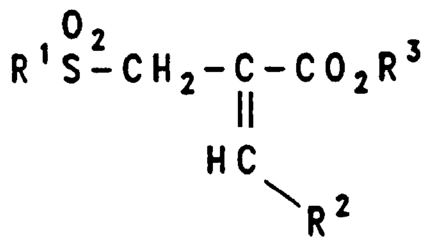

IX

$R^1$ und $R^2$ die zur Formel I und $R^3$ die zur Formel III angegebene Bedeutung haben
und
die Verbindungen der Formel IX überführt in Verbindungen der Verbindung I durch

a) alkalische Verseifung der Verbindungen der Formel IX zu Verbindungen der Formel XI,

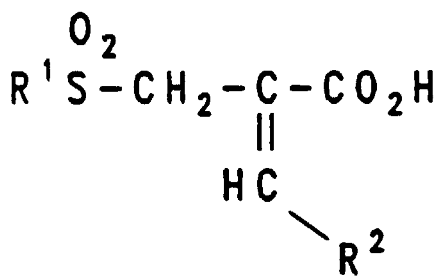

XI

worin $R^1$ und $R^2$ die zur Formel I angegebene Bedeutung haben und anschließende enantioselektive Hydrierung der Verbindungen der Formel XI zu Verbindungen der Formel I,
oder
b) enantioselektive Hydrierung der Verbindungen der Formel IX zu Verbindungen der Formel XII,

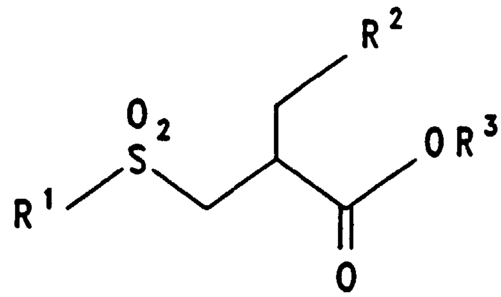

XII

worin $R^1$ und $R^2$ die zur Formel I und $R^3$ die zur Formel III angegebene Bedeutung haben und anschließende alkalische Verseifung der Verbindungen der Formel XII zu Verbindungen der Formel I.

Besonders geeignet ist das erfindungsgemäße Verfahren zur Herstellung von Verbindungen der Formel I worin

$R^1$ $C_1$-$C_4$-Alkyl, $C_5$-$C_6$-Cycloalkyl, ($C_6$-$C_{12}$)-Aryl-($C_1$-$C_3$)-alkyl oder $C_6$-$C_{12}$-Aryl bzw. Heteroaryl, die mit einer Hydroxy-, Methoxy- oder Trialkylsilyloxygruppe substituiert sein können; und
$R^2$ $C_6$-$C_{12}$-Aryl, das mit einer Methoxy-, Halogen-, Methyl-, Trifluormethyl-, oder Isopropylgruppe substituiert sein kann;
$C_3$-$C_6$-Heteroaryl, das mit einer Methoxy-, Halogen-, Methyl-, Trifluormethyl, oder Isopropylgruppe substituiert sein kann oder
$C_1$-$C_4$-Alkyl, -Alkenyl oder -Alkinyl, bedeuten.

Ganz besonders geeignet ist das erfindungsgemäße Verfahren zur Herstellung von Verbindungen der Formel I, worin

$R^1$ $C_1$-$C_4$-Alkyl,
($C_6$-$C_{12}$)-Aryl-($C_1$-$C_3$)-alkyl oder $C_6$-$C_{12}$-Aryl und
$R^2$ = $C_6$-$C_{12}$-Aryl, $C_3$-$C_6$-Heteroaryl oder $C_1$-$C_4$-Alkyl, -Alkenyl oder -Alkinyl bedeuten.

Weiterhin gehören zum Gegenstand der vorliegenden Erfindung Verbindungen der Formel XI

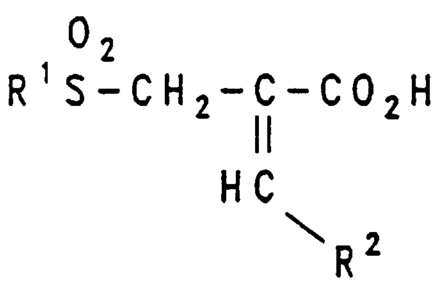

XI

worin $R^1$ und $R^2$ die in Anspruch 1 angegebene Bedeutung haben,

Ein weiterer Gegenstand der vorliegenden Erfindung sind die Verfahren zur Herstellung der Verbindungen der Formeln VIII, X, und XII in denen die Substituenten die in Anspruch 1 genannte Bedeutung haben gemäß den oben beschriebenen Verfahrensschritten.

Die Verbindungen der allgemeinen Formel I, VIII, X und XII können in der R- oder S-Form vorliegen.

Die Verbindungen der allgemeinen Formel VI, VII, IX und XI können als E (trans) oder Z (cis) Isomere vorliegen.

Die im erfindungsgemäßen Verfahren als Ausgangsmaterialien verwandten Mercaptane der Formel II sind kommerziell erhältlich (z. B. Fluka, Aldrich) bzw. nach literaturbekannten Verfahren (z. B. Organikum, Verlag VEB Berlin 1964, S. 176 und B. O. Isler et al. Helv. Chim Aeta 108, 903 (1956) oder nach R. P. Valante, Tetrahedron Lett. 22, 3119 (1981) herstellbar. Die entsprechenden Brendraubensäurederivate der Formel III können analog dem Verfahren von E. Cadis et al., CA 193 (21): 190763 g oder nach N. F. Blau, I. Org. Chem. 22, 83 (1957) hergestellt werden, sind aber

auch kommerziell erhältlich (z. B. Fluka).

Die anschließende S-Alkylierung der Mercaptane II mit den Verbindungen der Formel III zu der Verbindung der Formel IV erfolgt in Gegenwart von Basen, wie z. B. NaOH, KOH, NEt3, NaH, Natriumethylat vorzugsweise in einem organischen Lösungsmittel wie Tetrahydrofuran, Aceton, DME bei Temperaturen zwischen 0°C bis +70°C über 1 bis 6 Stunden. Eine bevorzugte Ausführungsform besteht in der Anwendung einer Zweiphasen-Katalyse in Wasser/Methylenchlorid in Gegenwart von $NaHCO_3$ als Base und einem Phasentranskatalysator.

Die Phosphorylide der Formel V werden z. B. in situ hergestellt aus den entsprechenden Phosphoniumsalzen in Gegenwart von Basen wie NaH, K-tert.butylat, Na-ethylat oder NaOH z. B. in einem Lösungsmittel wie z. B. Tetrahydrofuran, DME, DMSO bei Temperaturen von -10°C bis +60°C.

Die Umsetzung der Ylide V mit den Ketoverbindungen der Formel IV erfolgt in einer Wittigreaktion (Übersicht: B.E. Maryanoff, A.B. Reitz Phosphorus and Sulfur 27, 167 (1986) and Chem. Rev. 89, 863 (1989)) z. B. in Lösungsmitteln wie DMSO, Tetrahydrofuran, DME o. ä., bei Temperaturen von -10°C bis +80°C.

Zur Herstellung der erfindungsgemäßen Verbindungen der Formel VII werden die Verbindungen der Formel VI vorzugsweise in Lösungsmitteln, wie z. B. Alkoholen, DME/$H_2O$ oder dipolaren aprotischen Lösungsmitteln wie DMF, Acetonitril und DMSO gelöst und mit mehreren Äquivalenten $K_2CO_3$ oder NaOH versetzt und z. B. bei Temperaturen von 0°C bis +40°C 1 bis 2 Stunden verseift. Eine bevorzugte Ausführungsform besteht im Lösen in Methanol oder Äthanol und Behandlung mit 2 bis 4 Äquivalenten Kaliumcarbonat bei Zimmertemperatur über 2 Stunden.

Die Überführung von Verbindungen der Formel VI in Verbindungen der Formel VIII erfolgt mittels enantioselektiver Hydrierung.

Es ist bekannt, daß im Prinzip heterogene enantioselektive Hydrierungen mit einem Enantiomerenüberschuß von > 90 % ee an Raney-Nickel-Katalysatoren erzielt werden können, die mit Weinsäure und Natriumbromid modifiziert wurden (T. Harada et al., Chem. Lett. 1195 (1978); A. Tai et al., Chem. Lett. 2083 (1984)). Wegen des bekannten allergenen Potentials von Nickel und der schlechten Reproduzierbarkeit der Methode sind derartige heterogene Katalysatoren beispielsweise für die Synthese von Pharmaka jedoch nicht geeignet.

Die optische Induktion wird gemäß der vorliegenden Erfindung darum unter Verwendung eines gelösten, optisch reinen, metallorganischen Komplexes als Hydrierkatalysator erzielt. Der Katalysator braucht nur in niedriger Konzentration im Reaktionsgemisch vorhanden zu sein. Die molaren Verhältnisse von umgesetztem Substrat zu eingesetztem Katalysator liegen im allgemeinen je nach Katalysator und Substrat zwischen 100 und ca. 50000, vorzugsweise zwischen 100 und 5000. Die obere Grenze des Substrat/Katalysator-Verhältnisses ist im wesentlichen dadurch bedingt, daß der unter Hydrierbedingungen vorliegende "aktive Katalysator" in der Regel durch Sauerstoff rasch irreversibel verbraucht wird. Die benötigte Katalysatormenge hängt deshalb wesentlich davon ab, wie gründlich die Reaktionslösung vor Hydrierbeginn entgast werden kann und wie vollständig der Luftausschluß während der laufenden Hydrierung ist [siehe z. B. W. Vocke et al., Chem. Techn. 39, 123 (1987)].

In der Regel sind ca. 0,1 bis 0,5 Mol-% Katalysator ausreichend. Es kommen vorzugsweise Komplexe zur Anwendung, die als Zentralatom entweder ein Rhodium(I)-Kation oder ein Ruthenium(II)-Kation aufweisen.

Vor Hydrierbeginn wird vorzugsweise eine Lösung des Substrats, des Präkatalysators und gegebenenfalls von Additiven (z. B. eines Amins) in einem geeigneten inerten Lösungsmittel bzw. Lösungsmittelgemisch vorgelegt. Die entgaste Lösung wird dann unter 1 bis 500 atm, bevorzugt unter 1 bis 150 atm Wasserstoffgas geschüttelt oder gerührt.

Anstelle von Wasserstoffgas kann auch eine Transferhydrierung in Gegenwart einer Wasserstoffquelle, z. B. Ameisensäure/Triethylamin oder Isopropanol, zur Anwendung kommen.
Das Prinzip der enantioselektiven Transferhydrierung von C=C-Doppelbindungen wurde beschrieben von H. Brunner et al. [J. Organomet. Chem. 387, 209 (1990), Tetrahedron: Asymmetry 2, 331 (1991)].

Die Hydrierung kann in Abhängigkeit von Substrat und Katalysator z. B. im Temperaturbereich von etwa -40°C bis +200°C durchgeführt werden. Der Umfang der Hydrierung läßt sich durch Messung des Wasserstoffverbrauchs oder durch HPLC-Analyse verfolgen. Die empirisch zu ermittelnde Enantioselektivität der Hydrierung eines gegebenen Substrats wird von Parametern wie Reaktionstemperatur, Wasserstoffdruck, Natur des Lösungsmittels, Anwesenheit von Additiven in z. T. erheblichem Maße beeinflußt. Zum Beispiel geht die Enantioselektivität einiger optisch aktiver Rhodium(I)-diphosphin-Komplexe deutlich zurück, wenn ein bestimmter Wasserstoffgrenzdruck überschritten wird (siehe z. B. H. Takaheshi, K. Achiwa, Chem. Lett., 1921 (1987)), während beim Vorliegen eines andersartigen Diphosphins der Enantiomerenüberschuß (ee) des Produkts entweder unbeeinflußt bleibt (siehe z. B. U. Nagel et al., Chem Ber. 119, 3326 (1986)) oder sogar zunimmt (siehe z. B. A. Chan, WO 90/15790, Monsanto). Chan zeigte in Form umfangreicher Tabellen, daß bei der asymmetrischen Hydrierung von $\alpha$-Aryl-propionsäuren unter Verwendung verschiedener Ruthenium-BINAP-Katalysatoren die optische Reinheit des Hydrierprodukts stets mit steigendem Wasserstoffdruck und sinkender Reaktionstemperatur zunimmt. In der Regel nimmt die Hydriergeschwindigkeit mit steigendem Wasserstoffdruck zu und man benötigt weniger Katalysator. Auch eine Temperaturerhöhung führt in der Regel zu einer Beschleunigung der Hydrierung, ist jedoch häufig mit einer Erniedrigung des Enantiomerenüberschusses und einer geringeren Lebensdauer des Katalysators verbunden. In manchen Fällen wird jedoch das optimale Ergebnis durch

Temperaturerhöhung und Hydrierzeitverkürzung erreicht (siehe z. B. M. Kitamura et al., Tetrahedron Lett. 29, 1555 (1988)). Häufig kann man die Enantioselektivität der Hydrierung eines gegebenen Substrat/Katalysator-Paares durch Kühlung auf -20°C bis 0°C unter Inkaufnahme einer längeren Hydrierdauer verbessem (z. B. A. Chan., s. o.). Schwer berechenbar, aber z. T. wichtig, ist der Einfluß eines Additivs, wie z. B. Triethylamin oder optisch aktiven 1-Phenylethylamins, auf die Enantioselektivität der asymmetrischen Hydrierung. Während z. B. die Hydrierung von Phenylitaconsäuren mit dem Rhodiumkomplex von BPPM nur in Anwesenheit eines Äquivalents eines Amins zu Benzylbernsteinsäuren mit hohem Enantiomerenüberschuß führt, bleibt bei einigen anderen Substrat/Katalysator-Paaren der Enantiomerenüberschuß in Gegenwart von Triethylamin unbeeinflußt oder nimmt in wenigen Fällen sogar stark ab.

Ebenfalls empirisch zu bestimmen ist für ein gegebenes Metall (Rh' oder Ru") und ein gegebenes optisch aktives Diphosphin der Einfluß eines kationischen Präkatalysators gegenüber einem neutralen Präkatalysator. In der Mehrzahl der Fälle haben die kationischen Rhodium(I)-komplexe eine höhere katalytische Aktivität und eine größere Enantioselektivität als entsprechende neutrale Komplexe. In einigen Fällen liegen jedoch entgegengesetzte Verhältnisse vor.

Als Lösungsmittel für die asymmetrische Hydrierung sind im Prinzip alle Flüssigkeiten geeignet, die Substrat, Katalysator und eventuell zugesetzte Additive lösen und unter den Reaktionsbedingungen inert sind. Diese Voraussetzungen werden u. a. von unverzweigten und verzweigten Alkoholen erfüllt. Wegen der leichten Entfernbarkeit im Vakuum werden dabei Methanol, Ethanol, Propanol oder Isopropanol bevorzugt. Einige Katalysatoren zeigen jedoch eine mit fallender Lösungsmittelpolarität zunehmende Enantioselektivität, ergeben also in Isopropanol verglichen mit Methanol Produkte mit höherem Enantiomerenüberschuß. In diesen Fällen ist es zweckmäßig, ein mit dem Alkohol mischbares unpolares Co-Solvenz zuzusetzen bzw. die Hydrierung direkt in einem wenig polaren Lösungsmittel durchzuführen.

Eine Übersicht über bekannte chirale Diphosphine und die Verwendung von deren Rhodium(I) oder Rhodium (II)-Komplexen zur asymmetrischen Hydrierung von C=C-Doppelbindungen erhält man bei:

a) K. E. Koenig in "Asymmetric Synthesis", Vol. 5, Ed. Morrison, J. D. Academic Press, Orlando 1985, Seite 71 ff;
b) J. W. ApSimon et al., Tetrahedron 42, 5157 (1986), Seite 5173 bis 5186;
c) H. Brunner, Top. Stereochem. 18, 129 (1988);
d) I. Ojima et al., Tetrahedron 45, 6901 (1989), Seite 6902 bis 6916;
e) R. Noyori, H. Takaya, Acc. Chem. Res. 23, 345 (1990);
f) H. Takaya et al., Pure & Appl. Chem. 62, 1135 bis 1138 (1990);
g) M. J. Burk et al., Tetrahedron: Asymmetry 2, 569 bis 592 (1991).

Aus den angegebenen Übersichtsartikeln geht auch hervor, daß die chiralen Hydrierkatalysatoren in mannigfaltiger Weise modifiziert, z. B. durch Adsorption oder Bindung (evtl. über Spacer) an anorganischen Feststoffen (Kieselgel, Aktivkohle etc.) oder an quellfähigen Harzen (Polymeren) immobilisiert (siehe H. Brunner et al., J. Organomet. Chem. 384, 223 (1990)), oder durch geeignete hochpolare Substituenten in wasserlösliche Katalysatoren überführt werden können. Immobilisierte Katalysatoren können nach Reaktionsende durch Filtration aus dem Reaktionsgemisch entfernt werden, was eine Vereinfachung der Aufarbeitung darstellt. Außerdem können die abfiltrierten Katalysatoren gelegentlich ohne Aktivitäts- oder Enantioselektivitätsverlust in späteren Hydrierchargen wiederholt eingesetzt werden (Recycling). Der Einsatz wasserlöslicher Katalysatoren erlaubt die umweltfreundliche und billige Anwendung von Wasser als Lösungsmittel bzw. die einfach Entfernung des Katalysators nach Hydrierende durch Waschen mit Wasser bei Verwendung eines unpolaren Lösungsmittels. Die Verwendung derartiger modifizierter Hydrierkatalysatoren bei der asymmetrischen Hydrierung im Rahmen der enantioselektiven Synthese von Verbindungen der allgemeinen Formel 1 ist ebenfalls Gegenstand der vorliegenden Erfindung.

Ruthenium-Katalysatoren sind ökonomischer als Rhodium-Katalysatoren, da erstens ihr Preis auf molarer Basis nur ca. ein Fünftel beträgt und zweitens ihr nötiges Substrat/Katalysator-Verhältnis häufig günstiger ist. Möglichkeiten zur Rückgewinnung des Rhodiums nach Aufarbeitung der Hydrierlösungen bzw. zum Recycling des gesamten Katalysators wurden beschrieben (W. S. Karoles, Z. Chem. Education 63, 222 (1986); W. Vocke et al., Chem. Techn. 39, 123 (1987); O Cervinka et al., Czech CS 221, 487, CA 105; 9842 v).

Obwohl gemäß den Übersichsartikeln a) bis g) eine Vielzahl von Olefin-Strukturtypen asymmetrisch hydriert wurde, sind die im Rahmen der vorliegenden Erfindung durchgeführten asymmetrischen Hydrierungen der Sulfidcarbonsäure VI bzw. dessen Ester VII, sowie der Sulfoncarbonsäure XI bzw. dessen Ester IX bisher ohne Analogie. Die einzige literaturbekannte asymmetrische Hydrierung eines Substrats, welches ein Schwefelatom enthält, ist die durch chirale Rhodium(I)-diphosphin-Komplexe katalysierte Hydrierung

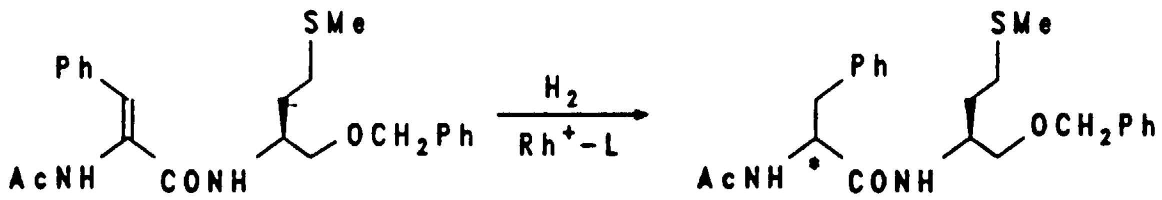


XIII XIV

(I. Ojima et al., Tetrahedron 40, 1255 bis 1268, dort Seite 1256 (1984)).

Dieser Stand der Technik unterscheidet sich in mehrfacher Weise grundlegend von den Substraten VI, VII, IX und XI der vorliegenden Erfindung.

1) Verbindung XIII ist als N-Acetyl-dehydroaminosäure-Derivat der "klassische", besterprobte Olefin-Strukturtyp für asymmetrische Hydrierungen basierend auf chiralen Rhodium-Katalysatoren

a) K. E. Koenig in "Asymmetric Synthesis", Vol. 5, Ed. Morrison, J. D. Academic Press, Orlando 1985, Seite 71 ff;
b) J. W. ApSimon et al., Tetrahedron 42, 5157 (1986), Seite 5173 bis 5186;
c) H. Brunner, Top. Stereochem. 18, 129 (1988);
d) I. Ojima et al., Tetrahedron 45, 6901 (1989), Seite 6902 bis 6916;
Die Substrate der vorliegenden Anmeldung gehören in Ermangelung einer Carbonylgruppe in Homoallyl-Position zu einem Olefin-Strukturtyp an dem bisher mit Rhodium-Katalysatoren keine brauchbaren optischen Induktionen erzielt werden konnte. $\alpha,\beta$-ungesättigte Carbonsäuren ohne andere komplexierungsfähige funktionelle Gruppen konnten zwar mit chiralen Ruthenium-Katalysatoren z. T. mit hoher optischer Induktion hydriert werden. Hier war jedoch unbekannt, ob derartige Katalysatoren schwefelhaltige Substrate tolerieren würden
e) R. Noyori, H. Takaya, Acc. Chem. Res. 23, 345 (1990);
f) H. Takaya et al., Pure & Appl. Chem. 62, 1135 bis 1138 (1990).

2) In Verbindung XIII befindet sich das Schwefelatom in einer Position, die die Ausbildung eines stabilen Rhodium (I)-chelats (im Zusammenwirken mit einer der beiden Carbonylgruppen oder der C=C-Doppelbindung) nicht zuläßt. XIII muß sich deshalb gegenüber dem Katalysator im Wesentlichen so verhalten; als sei die Schwefelfunktionalität nicht vorhanden. Die prinzipiell zur Komplexbildung fähige Sulfidgruppe der vorliegenden Substrate VI und VII bzw. die Sulfongruppe der vorliegenden Substrate IX und XI befindet sich jedoch genau in der Position, in der die klassischen Substrate die essentielle Carbonylgruppe haben. Die Ausbildung von Metallchelaten ist darum prinzipiell möglich deren Auswirkung auf Hydrierverlauf und optische Induktion nicht vorhersehbar.

3) Substrat XIII ist bereits homochiral. Die Stereoselektivität der Hydrierung setzt sich damit aus einer vom chiralen Katalysator bedingten Enantioselektivität und einer vom Substrat bedingten einfachen Diastereoselektivität zusammen. Die vorliegenden Substrate VI, VII, IX und XI sind allesamt prochiral; die bei der Hydrierung resultierende optische Induktion ausschließlich eine Katalysatoreigenschaft.

Das bisherige Fehlen literaturbekannter asymmetrischer Hydrierung von Alkenen mit Schwefelfunktionalität ist offensichtlich darin begründet, daß viele organische und anorganische Schwefelverbindungen, insbesondere Sulfide, bekannterweise sehr wirksame Katalysatorgifte für (trägergebundene) Edelmetall-Katalysatoren sind. Entsprechend erzielt man auch beim Einsatz von Rohprodukten der Substrate VI, VII, IX und XI (die bereits weitgehend $^{1}H$-NMR-rein sind) unter konventionellen Temperatur- und Wasserstoffdruck-Bedingungen mit chiralen Diphosphin-Rhodium- oder Ruthenium-Komplexen entweder keine Hydrierung, oder sie verläuft sehr langsam und unvollständig und führt zu Produkten sehr niedriger optischer Reinheit. Bei starker Erhöhung des Wasserstoffdrucks und (oder) der Temperatur, kann man dann häufig eine Hydrierung erzwingen, die aber durchweg mit sehr niedriger Enantioselektivität verläuft (siehe Beispiele).

Setzt man hingegen Substrate ein, die zum Beispiel durch mehrfache Umkristallisation, rigoros von Katalysatorgiften

befreit werden, so erzielt man bereits unter milden Bedingungen rasche, quantitative Hydrierungen, mit z. T. guten optischen Induktionen (siehe Beispiele).

Die Inhibition von Hydrierkatalysatoren durch Katalysatorgifte in rohen Hydriersubstraten kann im Rahmen der vorliegenden Erfindung in positiver Weise genutzt werden. Verbindungen der Formel VI fallen bei ihrer Herstellung durch Wittig-Reaktion in aller Regel als E/Z-Gemische an. Durch Wahl der konkreten Bedingungen der Wittig-Reaktion hat man einen begrenzten Einfluß auf das E/Z-Verhältnis, der zusammen mit der Wahl geeigneter Lösungsmittel für Umkristallisationen (z. B. bei VII) auch die Herstellung diastereomerenreiner E-Alkene erlaubt (siehe Beispiele).
Unter Bedingungen der Wittig-Reaktion, die im Technikum besonders leicht durchführbar sind, erhält man jedoch VI mit E/Z-Verhältnissen im Bereich 1 : 1 bis 2 : 1. In Abwesenheit von Reinigungen durch Umkristallisation, entstehen daraus auch die übrigen Hydriersubstrate VII, IX und XI mit entsprechenden E/Z-Verhältnissen.

Bei der asymmetrischen Hydrierung liegt eine ¶-Seitenselektivität vor, d. h. der chirale Katalysator unterscheidet Ober- und Unterseite der prochiralen Doppelbindung und überträgt den Wasserstoff ganz bevorzugt auf eine dieser beiden Seiten. Dabei geht ein E-Alken in ein Hydrierprodukt über, welches spiegelbildlich zum Hydrierprodukt des entsprechenden Z-Alkens ist. Weist ein Katalysator das nur theoretisch erreichbare Maximum von 100 % Enantioselektivität auf, so liefert er an einem diastereomerenreinen Substrat-Alken (nur E- oder nur Z-Konfiguration) ein Hydrierprodukt von 100 % ee. Liegt das gleiche Substrat-Alken hingegen als E/Z-Gemisch im Verhältnis 1 : 1 vor, so liefert der gleiche Katalysator bei vollständigem Hydrierverlauf ein racemisches Hydrierprodukt (0 % ee). Man erkennt, daß man zwecks Erzielung möglichst hoher optischer Reinheiten Hydriersubstrate möglichst hoher Diastereomeren-Reinheit benötigt. Wir haben überraschend gefunden, daß rohe Hydriersubstrate der Formeln VI, VII, IX und XI, die anfänglich ein E/Z-Verhältnis von 1 : 1 bis 2 : 1 aufweisen, unter den Bedingungen versuchter asymmetrischer Hydrierungen im Temperaturbereich von 20 bis 300°C, bevorzugt von 40 bis 150°C, besonders bevorzugt von 60 bis 120°C, weitgehende Z $\rightarrow$ E-Isomerisierungen ohne gleichzeitige Hydrierung erleiden. Auf diese Weise werden ein E/Z-Gemisch 70 : 30 der Sulfonsäure XIa mit einem neutralen Rhodium(I)-BPPM-Katalysator bei 120°C in Methanol unter 150 atm Wasserstoff innerhalb 20 Std. in 99 %iger Ausbeute in ein E/Z-Gemisch 97,1 : 2,9 der Sulfoncarbonsäure XIa umgewandelt. Auf analoge Weise wird bei 60°C die Sulfidcarbonsäure VIIa in 97,5 %iges E-Isomer von VIIa umgewandelt (siehe Beispiele).
Die auf diese Weise gewonnenen E-Isomeren sind nach Umkristallisation diastereomerenfrei (> 99,5 % E-Isomer).

Die Oxidation der Verbindungen der Formel VI zu den Verbindungen der Formel IX ist sowohl direkt als auch nach vorliegender Isolation der Sulfoxide möglich. Geeignete Oxidationsmittel sind z. B.:

Metachlorperbenzoesäure (mCPBA), z. B. in $CH_2Cl_2$ bei 0 bis 25°C;
Kaliumpermanganat ($KMO_4$), z. B. in $H_2O/K_2CO_3$ bei Raumtemperatur;
Oxone ($2KHSO_5 \bullet KHSO_4 \bullet K_2SO_4$) in Wasser bei Raumtemperatur;
Monoperoxyphthalsäure-Magnesiumsalz (MMPP) in Wasser bei Raumtemperatur;
$H_2O_2$, 30 %ig, bei Raumtemperatur in $CH_2Cl_2$, in Gegenwart von Essigsäure und Polyphosphorsäure, besonders bevorzugt ist $H_2O_2$.

Die Oxidation der Verbindungen der Formel VII, VIII und X zu den Verbindungen der Formel XI, XII, und I ist sowohl direkt als auch nach vorhergehender Isolation der Sulfoxide möglich. Als geeignete Oxidationsmittel kommen z. B. die bei der Oxidation der Verbindungen der Formel VI zu Verbindungen der Formel IX angewandten Reagenzien und Bedingungen in Frage.

Die alkalische Verseifung der Verbindungen der Formel VIII, IX und XII zu Verbindungen der Formel X, XI und I erfolgt unter analogen Bedingungen wie bei der Hydrolyse der Verbindungen der Formel VI zu den Verbindungen der Formel VII beschrieben.

Die Herstellung der chiralen Verbindungen der Formel X, XII und I erfolgt über eine enantioselektive Hydrierung der Verbindungen der Formel VII, IX und XI unter analogen Bedingungen wie bei der enantioselektiven Hydrierung der Verbindungen der Formel VI zu den Verbindungen der Formel VIII beschrieben.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels Kristallisation, Säulen-, Dünnschicht- oder Hochdruckflüssigkeitschromatographie.

Die Verbindungen der Formeln IV, VI und VII bis XII sind neu und stellen wertvolle Zwischenprodukte für die Herstellung von Verbindungen der Formel I dar. Die Erfindung betrifft daher auch diese Verbindungen sowie die Verfahren zu ihrer Herstellung wie vorstehend beschrieben.

Vorbemerkung: NMR-Spektren wurden, sofern nichts anderes angegeben, in $CDCl_3$ mit internem Standard TMS gemessen. Zur Klassifizierung von NMR-Signalen gelten folgende Abkürzungen:
s = Singulett, d = Dublett, p = Pentett, t = Triplett, q = Quartett.
Schmelzpunkte sind unkorrigiert

Durch die nachfolgenden Ausführungsbeispiele und durch den Inhalt der Patentansrüche soll die vorliegende

Erfindung näher erläutert werden.

Beispiel 1a

3-tert.-Butylthio-2-oxo-propionsäureethylester IVa ($R^1$ = t-Bu, $R^3$ = Et)

Zu einer Lösung von 42 g (0,5 Mol) Natriumhydrogencarbonat in 250 ml Wasser gibt man unter Rühren bei Raumtemperatur 10,6 g (25 Mmol) Methyl-trioctylammoniumchlorid (Hoechst AG) und 45 g (0,5 Mol) tert.-Butylmercaptan. Unter Kühlung wird bei max. 30°C eine Lösung von 97,5 g (0,5 Mol) Brombrenztraubensäureethylester (Fluka) in 400 ml Methylenchlorid innerhalb 1/2 Stunde unter Rühren zugetropft. Anschließend wird bei 20°C 2 1/2 Stunden weitergerührt. Die Methylenchlorid-Phase wird abgetrennt, mit $MgSO_4$ getrocknet, filtriert u. i. Vakuum eingeengt.
Ausbeute: 98 g (96 % d. Th.), hellgelbes Öl

$R_f$ = 0,67 (Cyclohexan/Essigester = 7:3)
MS: $C_8H_{14}SO_3$ m/e = 204
NMR: ($CDCl_3$): δ-Werte (Enolform, E/Z).
1.33 (s, t-$C_4H_9$), 1.36 (t, OEt), 1.45 (s, t-$C_4H_9$)
3.68 (s, $SCH_2$), 4.30 (q, OEt), 4.38 (q, OEt)
5.83 (d, -CH=), 6,53 (d, -CH=), 1.48 (s, OH)

Beispiel 2a

1-Naphthylmethyl-triphenyl-phosphoniumchlorid ($R^2$ = 1-Naphthyl)

340 g (1,93 Mol) 1-Chlormethyl-naphthalin (Aldrich) werden zusammen mit 505,7 g (1,93 Mol) Triphenylphosphin in 2 1 Acetonitril gelöst. Anschließend wird unter Rühren 5 Stunden am Rückfluß gekocht. Nach ca. 2 Stunden beginnt das Phosphoniumchlorid auszufallen. Man läßt abkühlen auf Raumtemperatur und saugt die Kristalle ab.
Ausbeute: 798 g (93,9 % d. Th.) weiße Krist, Fp: 308 bis 310°C

| | |
|---|---|
| Analyse: | C = 79, 8, H = 5,4, Cl = 8,3 gef. |
| | C = 79,0, H = 5,5, Cl = 8,1 ber. |

Beispiel 3a

1-tert.-Butylthiomethyl-1-ethoxycarbonyl-2-(1-naphthyl)-ethylen ($R^1$ = t-Bu, $R^2$ = 1-Naphthyl, $R^3$ = Et) VIa

In einem Vierhalskolben legt man unter Argon eine Suspension von 12,9 g (0,27 Mol) Natriumhydrid (55 %ige Suspension von Öl (Fluka)) in 500 ml abs. Tetrahydrofuran vor. Portionsweise gibt man unter Feuchtigkeitsausschluß innerhalb 1 Stunde 129,1 g (0,29 Mol) 1-Naphthylmethylentriphenyl-phosphoniumbromid (Beispiel 2a) unter Rühren zu. Nach beendeter Zugabe rührt man noch 1 Stunde bei Raumtemperatur. Die Reaktionsmischung wird dabei unter Bildung des Ylids Va ($R^2$ = 1-Naphthyl) dunkelrot. Zu der Ylidlösung werden dann 45.0 g (0,22 Mol) 3-tert.-Butylthio-2-oxo-propionsäureethylester IVa (Beispiel 1a) in 100 ml abs. Tetrahydrofuran zugetropft. Nach 4 Stunden Rühren bei Raumtemperatur wird der Reaktionsansatz unter Rühren zu einer Mischung von 500 ml gesättigter Natriumchloridlösung in Wasser und 500 ml Essigsäureethylester gegeben. Die organische Phase wird abgetrennt, die wäßrige Phase wird zur Entfemung von ausgefallenem Triphenylphosphinoxid filtriert und 1x mit 300 ml Essigester extrahiert. Die org. Extrakte werden vereinigt, mit etwas Aktivkohle und $MgSO_4$ versetzt, filtriert und i. Vak. eingeengt. Es werden 150 g (> 100 % d. Th.) braunrotes Öl erhalten. Das Rohprodukt (150 g) wird mit 500 ml Diethylester versetzt und über Nacht im Tiefkühlschrank bei 10°C aufbewahrt. Dann wird von dem ausgefallenen kristallinen Triphenylphosphinoxid abfiltriert und der Rückstand über eine 4 cm dicke Schicht aus Kieselgel filtriert. Das Filtrat wird im Vakuum eingeengt.
Ausbeute: 52 g (71 % d. Th.) hellgelbes Öl

$R_f$ = 0,80 (Cyclohexan/Essigester = 7 : 3)
MS: $C_{19}H_{22}SO_2$ m/e = 328 δ-Werte 0,74 (t, OEt); 1,42 (t, OEt); 1,28 und 1,45 (s, tBu); 3,58 und 3,73 (s, $SCH_2$); 3,90 und 4,48 (q, OEt); 7,25 bis 8,0 (m, aromat. Protonen, -CH=); 8,25 (s, -CH=) (E/Z-Gemisch im Verhältnis 1 : 1)

Beispiel 4a1

1-tert.-Butylthiomethyl-1-carboxy-2-(1-naphthyl)-ethylen ($R^1$ = t-Bu, $R^2$ = 1-Naphthyl, $R_3$ = H) VIIa

20 g (0,06 Mol) 1-tert.-Butylthiomethyl-1-ethyloxycarbonyl-2-(1-Naphthyl)-ethylen VIa (Beispiel 3a) werden in 200 ml Äthanol gelöst. Dann wird 120 ml 1N-NaOH zugegeben und 1 Stunde unter Rückfluß erwärmt. Man läßt abkühlen, versetzt mit 200 ml Essigester und 200 ml Wasser und stellt unter Rühren mit halbkonzentrierter Salzsäure auf pH = 3. Die org. Phase wird abgetrennt, die wäßrige Phase nach 2x mit 150 ml Essigester extrahiert. Die vereinigten org. Extrakte werden mit $MgSO_4$ getrocknet, filtriert, i. Vak. eingeengt.
Ausbeute: 17,0 g (92,8 % d. Th.)

$R_f$ = 0,13 (Cyclohexan/Essigsäureethylester = 7 : 3)
MS: $C_{17}H_{18}SO_2$ m/e: 300
NMR: ($CDCl_3$) δ-Werte: 1,29 und 1,46 (s, t-Bu);
3,58 und 3,75 (s, $SCH_2$); 7,8 bis 7,95 (m, aromat. Protonen); 8,25 und 8,42 (s, -CH=)
(E/Z-Gemisch im Verhältnis 1 : 1)

Beispiel 4a2

Herstellung eines reinen Diastereomeren von VIIa durch Umkristallisation des E/Z-Gemischs

14,3 g des E/Z-Gemischs aus Beispiel 4a1 werden mit 800 ml Cyclohexan versetzt. Mit einem auf 100°C vorgeheizten Ölbad wird das Gemisch 10 Min. unter einem Rückflußkühler erhitzt. Es entsteht eine fast klare Lösung, die heiß filtriert wird. Dabei werden < 100 mg tiefgelber Feststoff entfernt. Aus dem Filtrat kristallisiert beim 2-tägigen Stehen bei 0°C ein blaßgelber Feststoff, der abgesaugt und im Hochvakuum getrocknet wird.

Diese Umkristallisation wird zwei weitere Male wiederholt. Man erhält 4,8 g (67 % d. Th.) eines blaßgelben Feststoffs, Schmp. 167 bis 168°C, der gemäß HPLC < 0,5 % des Diasteromeren enthält.
NMR ($CDCl_3$) δ-Werte: 1,30 (s, t-Bu); 3,60 (s, $SCH_2$); 7,46 bis 7,63 (m, 3H, arom.-H); 7,84 bis 8,02 (m, 4H, arom.-H), 8,44 (s, 1H, =CH);
MS und DC wie oben beschrieben.

Beispiel 4a3

Herstellung eines reinen Diastereomeren von VIIa durch Z → E-Isomerisierung

Die Lösung von 3,0 g (10,0 mmol) des E/Z-Gemischs von VIIa in 100 ml Methanol wird mittels durchperlendem Argon entgast. 10 mg (0,02 mmol) Chloro(1,5-Cyclooctadien-rhodium(I)dimer (Fluka), 27,7 mg (0,05 mmol)(2S,4S)-(-)-N-tert.-Butoxycarbonyl-4-diphenylphosphino-2-diphenylphosphinomethylpyrrolidin ((-)-BPPM, Merck-Schuchardt) und 1,11 g (11,0 mmol) Triethylamin werden unter Argon zugegeben. Man schüttelt das Reaktionsgemisch 16 Stunden bei 60°C unter 50 atm Wasserstoff. HPLC-Analyse des Reaktionsgemisches unter den Bedingungen von Beispiel 7a ergibt 97,5 % (E)-VIIa, 2 % (Z)-VIIa und 0,5 % Hydrierprodukt X. Aufarbeitung gemäß Beispiel 7a ergibt 2,9 g (97 % d. Th.) (E)-VIIa. Umkristallisation dieses Produkts gemäß Beispiel 4a2 gibt 2,4 g (80 % d. Th. bez auf eingesetztes E/Z-VIIa) (E)-VIIa, das gemäß HPLC < 0,5 % des Diasteromeren enthält. Dieses Produkts ist bezüglich Schmp. und $^1$H-NMR identisch mit dem aus Beispiel 4a2.

Beispiel 5a

3-tert.-Butylthio-2(S)-(1-naphthylmethyl)-propionsäureethylester ($R^1$ = t-Bu, $R^2$ = 1-Naphthyl, $R^3$ = Et) VIII

dieses Beispiel muß zu einem späterem Zeitpunkt nachgeliefert werden

Beispiel 6a

1-tert.-Butylsuflonylmethyl-1-ethoxycarbonyl-2-(1-naphthyl)-ethylen ($R^1$ = t-Bu, $R^2$ = 1-Naphthyl, $R^3$ = Et) IXa

16 g (48 mMol) 1-tert.-Butylthiomethyl-1-ethoxycarbonyl 2-(1-naphthyl)-ethylen VIa (Beispiel 3a) werden in 160 ml Essigester und 9,6 ml (160 mMol) Eisessig gelöst. Bei Raumtemperatur werden unter Rühren 16 ml (160 mMol) 30 %iges Wasserstoffperoxid zugetropft. Dann wird noch 3 Stunden bei Raumtemperatur gerührt, mit 200 ml Wasser

und 200 ml Essigester versetzt. Die org. Phase wird abgetrennt, mit Natriumhydrogencarbonat-Lösung wird säurefrei gewaschen, mit $MgSO_4$ getrocknet, i. Vak. eingeengt.
Ausbeute: 14 g (83 % d. Th) weiße Kristalle, Fp = 94 bis 98°C

$R_f$ = 0,28 und 0,38 (Cyclohexan/Essigester = 7 : 3)
MS: $C_{20}H_{24}SO_4$ m/e 360
NMR: ($CDCl_3$) δ-Werte: 1,28 und 1,43 (t, OEt), 1,36 und 1,54 (s, t-Bu); 3,90 und 4,41 (q, OEt); 4,21 und 4,28 (s, $CH_2CO_2$); 7,30 bis 7,05 (m, aromatische Protonen); 7,92 und 8,58 (s, -CH=)
(E/Z-Gemisch im Verhältnis 1 : 1)

Beispiel 7a

3-tert.-Butylthio-2-(S)-(1-naphthylmethyl)-propionsäure ($R^1$ = t-Bu, $R^2$ = 1-Naphthyl, $R^3$ = H) X

3,0 g (10 mmol) 1-tert.-Butylthiomethyl-1-carboxy-2-(E)-(1-naphthyl)-ethylen VIIa (Beispiel 4a, < 0,5 % des Z-Isomeren) werden in einem Glaseinsatz für einen Stahlautoklaven in 500 ml Methanol gelöst und mittels durchperlendem Argon entgast. 621 mg (0,1 mmol) (S)-(-)-2,2'-Bis-(diphenylphosphino)-1,1'-binaphthalin ((S)-(-)-BINAP, Strem Chemicals) und 26 mg (0,05 mmol) Benzol-ruthenium(II)-chlorid dimer (Aldrich) werden in einen anderen Gefäß unter Argon in 50 ml Dimethylformamid gelöst, dann 10 Minuten auf 100°C erhitzt und wieder auf 25 °C abgekühlt, stets unter Argon. Eine Argon-entgaste Lösung von 171 mg (30,8 mmol) Natriumacetat in 20 ml Methanol wird hinzugegeben, 5 Minuten bei 25°C gerührt und die entstehende Katalysatorlösung unter Argon zu der Substratlösung im Glaseinsatz gegossen. Der Glaseinsatz wird in einen Argon-gefüllten Autoklaven eingelegt. Die Argon-Atmosphäre wird durch Wasserstoff verdrängt. Die Reaktionslösung wird bei 120°C 20 Stunden unter 150 atm Wasserdruck geschüttelt. Man spült mit Stickstoff, entnimmt den Glaseinsatz, entfernt das Lösungsmittel im Vak. und nimmt den Rückstand in 300 ml Methyl-tert.-butylether auf. Man schüttelt 2 Minuten mit 100 ml 0,5N Salzsäure. Die organische Phase wird abgetrennt und die wäßrige Phase mit 100 ml MTB-Ether extrahiert. Die vereinigten organischen Phasen werden mit 50 ml 0,5N Salzsäure, 50 ml Wasser gewaschen, über Magnesiumsulfat getrocknet. Das Lösungsmittel wird im Vak. entfernt und das zurückbleibende Öl im Hochvakuum getrocknet. Das Produkt (3,01 g, 9,95 mmol, 99,5 % d. Th.) hat gemäß HPLC {250 x 4,00 mm ®Nuceosil 100 C18 7 µm, Detektor 300 nm, 1,5 ml/Min [51 % Waser/49 % Acetonitril + 0,05 % $NH_4H_2PO_4$ mit 85 %iger Phosphorsäure auf pH = 3,5 gestellt], {$t_{ret}$: (Z)-VII 15,2 Min), X 16,8 Min, (E)-VIIa 18,50 Min} eine Reinheit von 98 %. $[\alpha]_D^{25}$ = 2,30° (c = 1,06, Derivatisierung mit MEPA/optisch reinem (R)-(+)-1-Phenylethylamin und anschließende HPLC-Trennung auf RP18-®LiChrospher 100 mit Acetonitril/Wasser 1 : 1 ergibt die Peaks der beiden diastereomeren Amide im Verhältnis 85 : 15. (S)-X hat somit 70 % ee.

Beispiel 8a

Die ungesättigte (E)-Sulfidcarbonsäure VIIa wird durch eine Reihe weiterer asymmetrischer Hydrierungen in die gesättigte (S)-Sulfidcarbonsäure (S)-X überführt. Hydrierbedingungen, sowie die jeweilige Ausbeute und optische Reinheit des Hydrierprodukts können der Tabelle 1 entnommen werden

Beispiel 8a

Tabelle 1: Asymmetrische Hydrierungen der Sulfidcarbonsäure der Formel VIIa zu Xa

| Katalysator Formel | isoliert/ in situ erzeugt | Substrat/ Katalysator-verhältnis (mmol/mmol) | $H_2$-Druck (bar) | Temp. (°C) | Hydrierzeit (Std.) | Umsatz (%)[a] | optische Reinheit von Xa (%ee) |
|---|---|---|---|---|---|---|---|
| 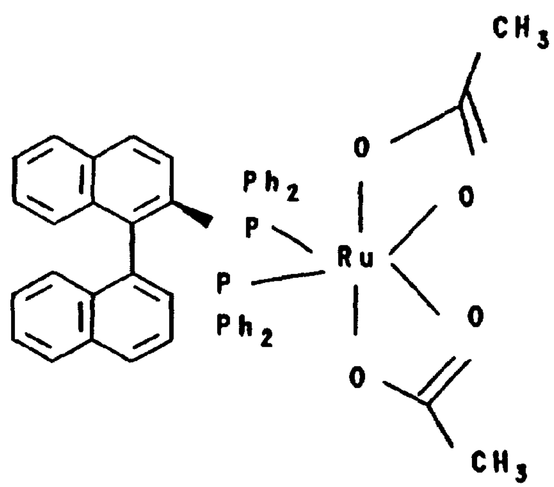 | isoliert | 100 | 150 | 150 | 1/6 | 30 | 43 |
| | isoliert | 100 | 150 | 150 | 1 | 100 | 65 |
| | isoliert | 100 | 150 | 120 | 5 | 100 | 70 |
| | in situ | 100 | 150 | 120 | 20 | 100 | 72 |
| | isoliert | 100 | 150 | 80 | 6 | 100 | 72 |
| | isoliert | 100 | 150 | 50 | 48 | 100 | 39 |
| | isoliert | 100 | 150 | 25 | 96 | 100 | 54 |
| | isoliert | 100[b] | 150 | 50 | 48 | 100 | 0 |

| Katalysator Formel | isoliert/ in situ erzeugt | Substrat/ Katalysator-verhältnis (mmol/mmol) | $H_2$-Druck (bar) | Temp. (°C) | Hy-drier-zeit (Std.) | Umsatz (%)[a] | optische Reinheit von Xa (%ee) |
|---|---|---|---|---|---|---|---|
| 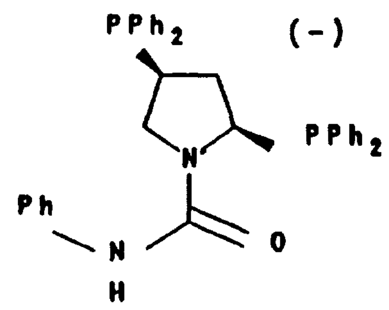 $[Rh(COD)_2]^+ BF_4^-$ | in situ | 200 | 1 | 20 | 24 | | 0 |
| | | 200 | 100 | 20 | 20 | 17 | 52 |
| | | 100 | 30 | 50 | 20 | 98 | 43 |
| | | 100 | 150 | 50 | 20 | 99 | 48 |
| | | 50 | 150 | 120 | 20 | 100 | 46 |
| 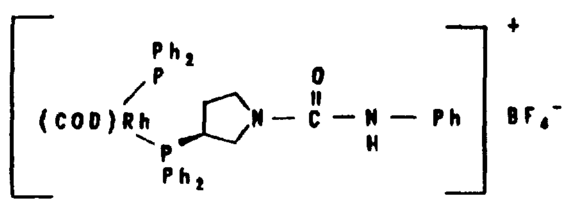 | isoliert | 50 | 150 | 120 | 20 | ~ 100[c] | 4 |

a) % Produkt Xa bezogen auf Summe von % Produkt Xa und % Ausgangsmaterial VIIa
b) in Gegenwart von 1.05 Äquivalenten (bezogen auf VIIa) Triethylamin
c) Das Ausgangsmaterial VIIa ist vollständig verschwunden. Neben 80 % Xa entstehen 20 % Verunreinigungen.

Beispiel 9a

1-tert.-Butylsulfonylmethyl-1-carboxy-2-(1-naphthyl)-ethylen ($R^1$ = t-Bu, $R^2$ = 1-Naphthyl, $R^3$ = H) XIa

17 g (56 mMol) 1-tert.-Butylthiomethyl-1-carboxy-2-(1-naphthyl)-ethylen VIIa (Beispiel 4a) werden in 170 ml Essigsäureethylester und 10 ml Eisessig gelöst. Bei Raumtemperatur werden 17 ml (170 mMol) 30 %iger Wasserstoffperoxid unter Rühren zugetropft. Dann wird noch 5 Stunden bei Raumtemperatur nachgerührt, mit 200 ml Essigester versetzt und die org. Phase 5x mit je 100 ml gesättigter Kochsalzlösung extrahiert. Die org. Phase wird abgetrennt, mit $MgSO_4$ getrocknet, i. Vakuum eingeengt, ggf. nochmals mit 100 ml Essigester versetzt und erneut i. Vakuum einrotiert um Reste von Eisessig zu entfernen. Der Rückstand wird mit 90 ml Methyl-tert.-Butyl-ether versetzt und im Tiefkühlschrank über Nacht kristallisiert.
Ausbeute: 14,8 g (80 % d. Th.) hellgelbe Kristalle Fp. 80°C

$R_f$ = 0,30 und 0,52 (Cyclohexan/Essigester/AcOH = 20 : 80 :1)
MS: $C_{18}H_{20}SO_4$ m/e = 332
NMR: ($CDCl_3$) δ-Werte: 1,33 und 1,55 (s, t-Bu); 4,20 und 4,28 (s, $CH_2SO_2$); 7,4 bis 8,1 (m, aromat. Protonen), 7,48 und 8,73 (s, -CH=)
(E/Z-Gemisch im Verhältnis 1 : 1)

Entfernung von Katalysatorgiften durch Umkristallisation:

11,9 g der obigen rohen Carbonsäure (E/Z ~ 2 : 1) wurden mit 400 ml n-Heptan versetzt und zum Rückfluß erhitzt. In die siedende Suspension wird portionsweise durch den Rückflußkühler Methyl-tert.-Butylether zugegossen. Nach Zugabe von 200 ml MTB-Ether entsteht eine fast klare Lösung. Geringe Mengen (< 100 mg) Feststoff werden heiß abgesaugt und verworfen. Man läßt das Filtrat langsam auf Raumtemperatur abkühlen (weiterhin klare Lösung), versetzt mit ca. 20 mg Impfkristallen und kühlt auf 0°C, nach einigen Stunden dann für einen Tag auf -20°C. Die Kristalle werden abgesaugt, mit 2x 100 ml n-Heptan gewaschen und 2 Tage im Hochvakuum getrocknet. Man erhält 8,0 g weiße Kristalle, die gemäß $^1H$-NMR ein E/Z-Verhältnis von ~ 2 : 1 aufweisen und 0,5 Mol MTB-Ether enthalten, Schmp. 96 bis 105°C. Weiteres Trocknen im HV bei 80°C für 8 Stunden gibt 7,0 g graue Kristalle, die keinen MTB-Ether mehr enthalten, Schmp. 100 bis 115°C.

Beispiel 10a

1-tert.-Butylsulfonylmethyl-1-carboxy-2-(1-naphthyl)-ethylen ($R^1$ = t-Bu, $R^2$ = 1-Naphthyl, $R^3$ = H) XIa

13 g (36 mMol) 1-tert.-Butylsulfonylmethyl-1-ethyloxycarbonyl-2-(1-naphthyl)ethylen IXa (Beispiel 6a) werden in 130 ml Ethanol gelöst Dann werden 80 ml 1 N Natronlauge zugegeben und 1 1/2 Stunden bei 50°C gerührt Es wird abgekühlt, mit 200 ml Essigester, 200 ml Wasser versetzt und mit halbkonzentrierter Salzsäure versetzt, die Wasserphase nochmals mit 200 ml Essigester extrahiert. Die vereinigten organischen Phasen werden mit $MgSO_4$ getrocknet, filtriert und im Vakuum eingeengt. Der Rückstand (15 g) wird aus 50 ml Methyl-tert.-Butylether umkristallisiert und über Nacht im Tiefkühlschrank aufbewahrt.
Ausbeute: 11,6 g (96 % d. Th.) hellgelbe Kristalle Fp. 80°C

$R_f$ = 0,30 und 0,52 (Cyclohexan/Essigester/AcOH = 20 : 80 : 1)
MS: $C_{18}H_{20}SO_4$ m/e = 332
NMR: ($CDCl_3$) δ-Werte: analog Bsp. 8a
(E/Z-Gemisch im Verhältnis 1 : 1)

Beispiel 11a

3-tert.-Butylsulfonyl-2(S)-(1-naphthylmethyl)propionsäure ($R^1$ = t-Bu, $R^2$ = 1-Naphthyl, $R^3$ = H) I durch Oxidation von X

681 mg (2,25 mmol) 3-tert.-Butylthio-2(S)-(1-naphthylmethyl)-propionsäure von 70 % ee (Beispiel 7a) werden in 450 µl Eisessig und 1,35 ml Dichlormethan gelöst. Unter Eiskühlung werden 0,75 ml (8,7 mmol) 35 %ige wäßrige

Wasserstoffperoxid-Lösung zugegeben und 1 Stunde bei 0°C gerührt. HPLC zeigt fast vollständigen Umsatz des Ausgangsmaterials unter Bildung von überwiegend Sulfoxid und wenig Sulfon.
10 g Polyphosphorsäure werden in 10 g 85 %iger Ortho-Phosphorsäure gelöst und von dieser Lösung 200 µl der obigen Reaktionslösung zugesetzt und noch 3 Stunden bei Raumtemperatur gerührt. HPLC zeigt vollständige Oxidation des Sulfoxids zum Sulfon. 10 ml Dichlormethan und 10 ml Wasser werden zugegeben und durchgeschüttelt. Die organische Phase wird abgetrennt und die wäßrige Phase mit 2x 10 ml Dichlormethan extrahiert. Die vereinigten organischen Phasen werden mit 3x 5 ml 10 %iger Natirumbisulfitlösung, dann mit 2x 3 ml Wasser gewaschen, über Magnesiumsulfat getrocknet, filtriert. Das Lösungsmittel wird im Vakuum entfernt und der Rückstand im HV getrocknet. Man erhält 680 mg (90 % d. Th.) eines blaßgelben, festen Schaums, $[\alpha]_D^{25}$ = + 1,19° (c = 0,98, $CH_3OH$). Derivatisierung mit MEPA/optisch reinem (R)-(+)-1-Phenylethylamin und anschließende HPLC-Analyse wie bei Beispiel 7a ergibt 74 % ee.
Die Oxidation ist also mit keinerlei Minderung der optischen Reinheit verbunden
MS: $C_{18}H_{22}SO_4$ m/e = 334
NMR ($CDCl_3$) δ-Werte: 1,27 (s, t-Bu); 3,00 bis 3,18 (m, 1H, $CH\text{-}CO_2H$); 3,35 bis 3,80 (m, 4H, $2xCH_2$); 5,40 bis 6,70 (sehr breites s, 1H, $CO_2H$); 7,33 bis 7,65 (m, 4H, arom.-H); 7,75 bis 7,90 (ddd, 2H, arom.-H); 8,13 (d, 1H, arom.-H).
550 mg dieses Produkts von 74 %ee werden in 5 ml Toluol/MTB-Ether 7:3 glatt
gelöst. Man läßt die Lösung 4 Tage bei Raumtemperatur in einem Kolben stehen, der eine langsame Lösungsmittel-Verdunstung zuläßt. In dem angebenen Zeitraum verdunstet etwa die Hälfte des Lösungsmittels und das Produkt kristallisiert in Form großer Plättchen. Die Mutterlauge wird im Vakuum zur Trockne eingeengt.

| | |
|---|---|
| Kristalle: | 170 mg, 16 %ee |
| aus Mutterlauge: | 370 mg, 96 %ee. |

Beispiel 12a

3-tert.-Butylsulfonyl-2(S)-(1-napthylmethyl)-propionsäure ($R^1$ = t-Bu, $R^2$ = 1-Naphthyl, $R^3$ = H) I durch Verseifung von XII

Die Verseifung erfolgt völlig analog zu Beispiel 10a.
Derivatisierung mit MEPA/optisch reinem (R)-(+)-1-Phenylethylamin und anschließende HPLC-Analyse wie bei Beispiel 7a belegt, daß die Verseifung mit keinerlei Minderung der optischen Reinheit verbunden ist.
Die Spektren des Produkts entsprechen denen aus Beispiel 11a.

## Patentansprüche

1. Verfahren zur stereoselektiven Herstellung einer Verbindung der Formel I

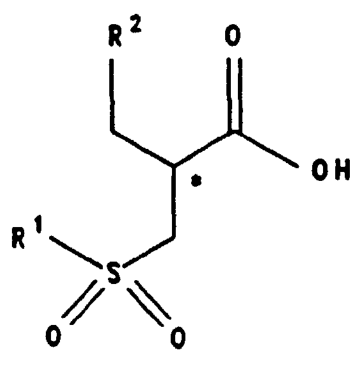

I

worin

$R^1$ $C_1$-$C_6$-Alkyl, $C_3$-$C_6$-Cycloalkyl, ($C_6$-$C_{12}$)-Aryl-($C_1$-$C_4$)-alkyl oder $C_6$-$C_{12}$-Aryl bzw. -Heteroaryl bzw. -Heterocycloalkyl, die mit 1, 2 oder 3 gleichen oder verschiedenen Hydroxy-, Methoxy- oder Trialkylsilyloxygruppen substituiert sein können; und
$R^2$ $C_6$-$C_{12}$-Aryl, das mit 1, 2 oder 3 gleichen oder verschiedenen Methoxy-, Halogen-, Cyano-, Methyl-, Trifluormethyl-, Isopropyl- oder Nitrogruppen substituiert sein kann;

$C_3$-$C_9$-Heteroaryl, das mit 1, 2 oder 3 gleichen oder verschiedenen Methoxy-, Halogen-, Cyano-, Methyl-, Trifluormethyl-, Isopropyl- oder Nitrogruppen substituiert sein kann; oder
$C_1$-$C_{10}$-Alkyl, Alkenyl oder Alkinyl, die durch 1, 2 oder 3 gleiche oder verschiedene Methoxy-, Halogen-, Cyano-, Methyl-, Trifluormethyl-, Isopropyl- oder Nitrogruppen substituiert sein können, bedeuten

und die Verbindungen der Formel I in der R- oder S-Form vorliegen können, dadurch gekennzeichnet, daß man

I) Mercaptane der Formel II

$$R^1\text{-SH} \qquad \text{II}$$

wobei $R^1$ die zur Formel I angegebene Bedeutung hat, in Gegenwart einer Base umsetzt mit einer Verbindung der Formel III

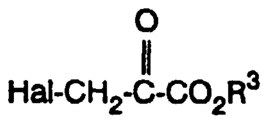

III

wobei Hal = Jod, Brom oder Chlor sein kann und $R^3$ ein geradkettiger oder verzweigter Alkylrest mit 1 bis 6 C-Atomen sein kann, zu einer Verbindung der Formel IV

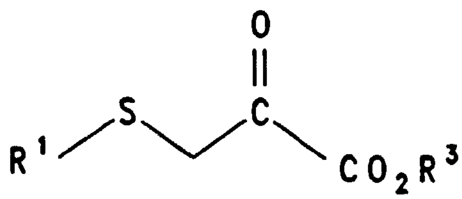

IV

wobei $R^1$ die zu Formel I und $R^3$ die zu Formel III angegebene Bedeutung haben, und

II) die Verbindungen der Formel IV mit Yliden der Formel V

$$R^2\text{-CH=P}(C_6H_5)_3 \qquad \text{V}$$

wobei $R^2$ die zur Formel I gegebene Bedeutung hat, umsetzt zu den ungesättigten Verbindungen der Formel VI

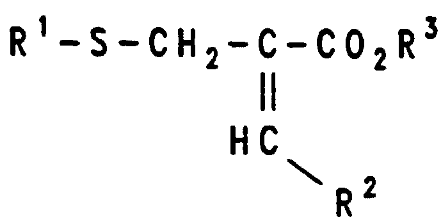

VI

wobei $R^1$ und $R^2$ die zu Formel I und $R^3$ die zu Formel III angegebene Bedeutung haben, und

III) die Verbindungen der Formel VI überführt in Verbindungen der Formel I indem man

A) die Verbindungen der Formel VI alkalisch verseift zu Verbindungen der Formel VII,

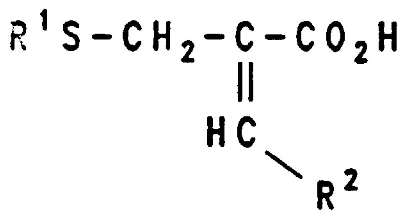

VII

worin $R^1$ und $R^2$ die zur Formel I angegebene Bedeutung haben,
und durch

a) enantioselektive Hydrierung Verbindungen der Formel VII in Verbindungen der Formel X überführt

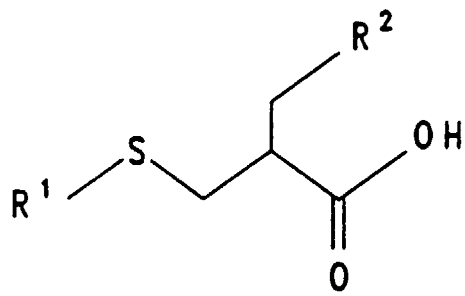

X

worin $R^1$ und $R^2$ die zur Formel I angegebene Bedeutung haben, und durch anschließende Oxidation Verbindungen der Formel X in Verbindungen der Formel I überführt
oder
b) durch Oxidation Verbindungen der Formel VII zu Verbindungen der Formel XI umsetzt

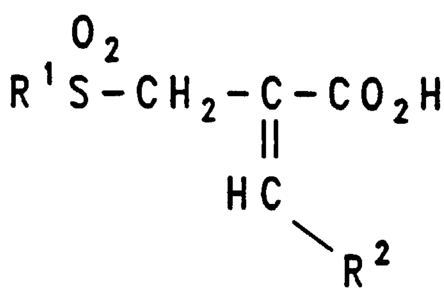

XI

worin $R^1$ und $R^2$ die zur Formel I angegebene Bedeutung haben, und durch anschließende enantioselektive Hydrierung der Verbindungen der Formel XI Verbindungen der Formel I erhält,
oder

B) die Verbindungen der Formel VI mit chiralen Katalysatoren enantioselektiv hydriert zu den Verbindungen der Formel VIII

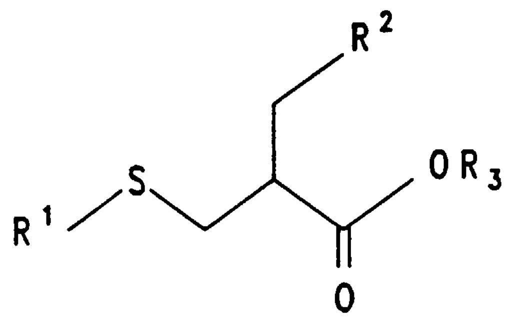

VIII

worin $R^1$ und $R^2$ die zur Formel I und $R^3$ die zur Formel III angegebene Bedeutung haben, und durch

a) alkalische Verseifung Verbindungen der Formel VIII in Verbindungen der Formel X

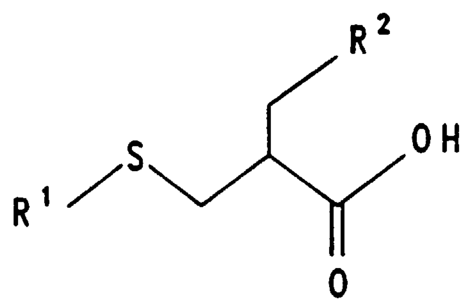

X

worin $R^1$ und $R^2$ die zur Formel I angegebene Bedeutung haben, überführt und durch anschließende Oxidation der Verbindungen der Formel X Verbindungen der Formel I erhält,
oder
b) durch Oxidation der Verbindungen der Formel VIII Verbindungen der Formel XII erhält,

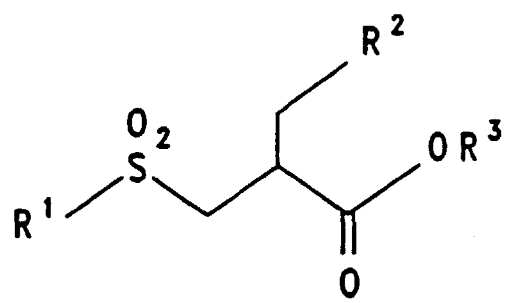

XII

worin $R^1$ und $R^2$ die zur Formel I und $R^3$ die zur Formel III angegebene Bedeutung haben und anschließend durch alkalische Verseifung Verbindungen der Formel XII in Verbindungen der Formel I überführt,
oder

C) die Verbindungen der Formel VI oxydiert zu den Verbindungen der Formel IX, wobei

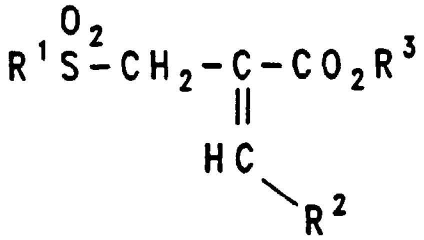

IX

$R^1$ und $R^2$ die zur Formel I und $R^3$ die zur Formel III angegebene Bedeutung haben und
die Verbindungen der Formel IX überführt in Verbindungen der Verbindung I durch

a) alkalische Verseifung der Verbindungen der Formel IX zu Verbindungen der Formel XI,

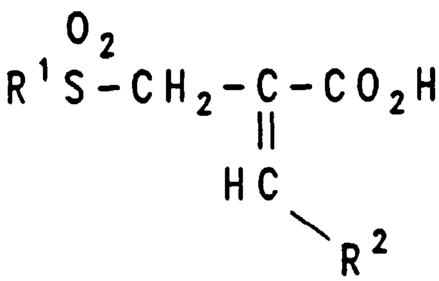

XI

worin $R^1$ und $R^2$ die zur Formel I angegebene Bedeutung haben und anschließende enantioselektive Hydrierung der Verbindungen der Formel XI zu Verbindungen der Formel I,
oder
b) enantioselektive Hydrierung der Verbindungen der Formel IX zu Verbindungen der Formel XII,

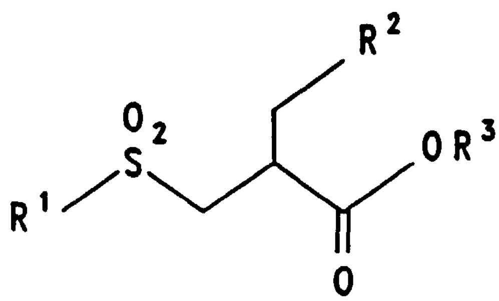

XII

worin $R^1$ und $R^2$ die zur Formel I und $R^3$ die zur Formel III angegebene Bedeutung haben und anschließende alkalische Verseifung der Verbindungen der Formel XII zu Verbindungen der Formel I.

**2.** Verfahren gemäß Anspruch 1, dadurch gekennzeichnet, daß

$R^1$ $C_1$-$C_4$-Alkyl, $C_5$-$C_6$-Cycloalkyl, ($C_6$-$C_{12}$)-Aryl-($C_1$-$C_3$)-alkyl oder $C_6$-$C_{12}$-Aryl bzw. Heteroaryl, die mit einer Hydroxy-, Methoxy- oder Trialkylsilyloxygruppe substituiert sein können; und
$R^2$ $C_6$-$C_{12}$-Aryl, das mit einer Methoxy-, Halogen-, Methyl-, Trifluormethyl-, oder Isopropylgruppe substituiert sein kann;
$C_3$-$C_6$-Heteroaryl, das mit einer Methoxy-, Halogen-, Methyl-, Trifluormethyl, oder Isopropylgruppe substituiert sein kann oder
$C_1$-$C_4$-Alkyl, -Alkenyl oder -Alkinyl, bedeuten.

**3.** Verfahren gemäß den Ansprüchen 1 oder 2, dadurch gekennzeichnet, daß

$R^1$ $C_1$-$C_4$-Alkyl,
($C_6$-$C_{12}$)-Aryl-($C_1$-$C_3$)-alkyl oder $C_6$-$C_{12}$-Aryl und
$R^2$ = $C_6$-$C_{12}$-Aryl, $C_3$-$C_6$-Heteroaryl oder $C_1$-$C_4$-Alkyl, -Alkenyl oder -Alkinyl bedeuten.

**4.** Verfahren zur Herstellung von Verbindungen der Formel X

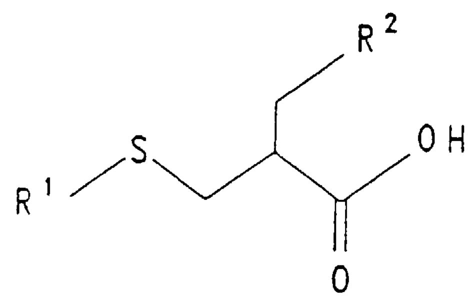

X

durch enantioselektive Hydrierung einer Verbindung der Formel VII

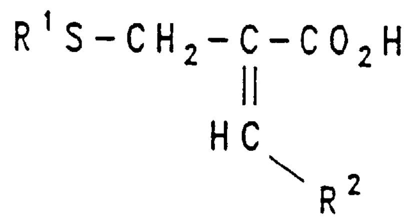

VII

worin $R^1$ und $R^2$ die zu Formel I genannten Bedeutungen haben.

**5.** Verfahren zur Herstellung von Verbindungen der Formel VIII

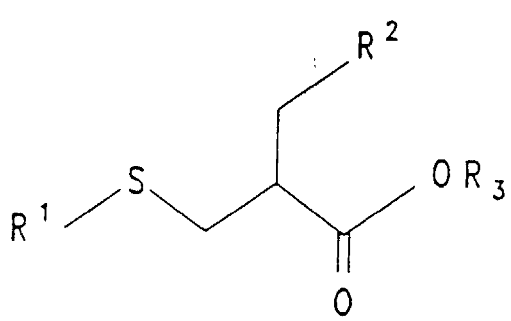

VIII

durch enantioselektive Hydrierung einer Verbindung der Formel VI

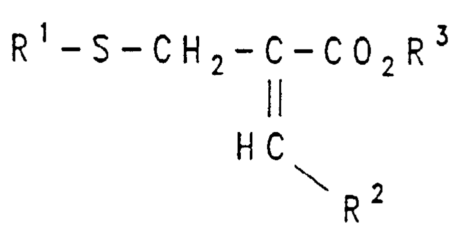

VI

worin $R^1$ und $R^2$ die zu Formel I genannten Bedeutungen haben und $R^3$ die zu Formel III genannten Bedeutungen

hat.

**6.** Verfahren zur Herstellung von Verbindungen der Formel XII

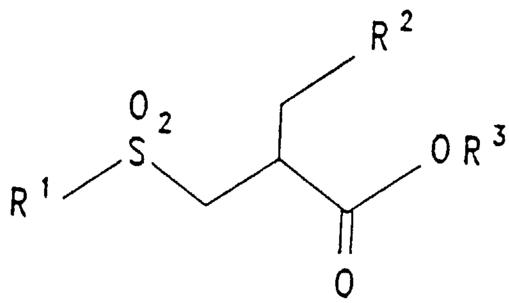

XII

durch enantioselektive Hydrierung einer Verbindung der Formel IX

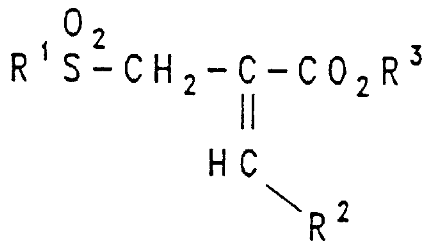

IX

worin $R^1$ und $R^2$ die zu Formel I genannten Bedeutungen haben und $R^3$ die zu Formel III genannten Bedeutungen hat.

**7.** Verbindungen der Formel XI

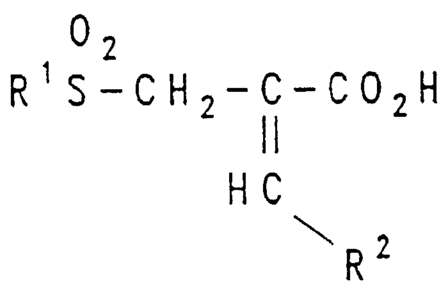

XI

worin $R^1$ und $R^2$ die in Anspruch 1 angegebene Bedeutung haben.

**Claims**

**1.** A process for the stereoselective preparation of a compound of the formula I

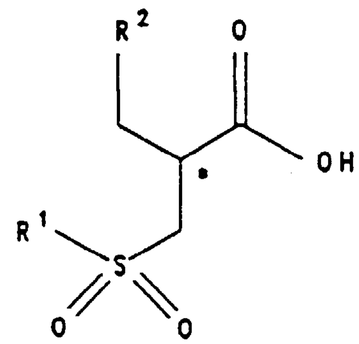

I

in which

$R^1$ is $C_1$-$C_6$-alkyl, $C_3$-$C_6$-cycloalkyl, ($C_6$-$C_{12}$)-aryl-($C_1$-$C_4$)-alkyl or $C_6$-$C_{12}$-aryl or -heteroaryl or -heterocycloalkyl, which may be substituted by 1, 2 or 3 identical or different hydroxyl, methoxy or trialkylsilyloxy groups; and
$R^2$ is $C_6$-$C_{12}$-aryl which may be substituted by 1, 2 or 3 identical or different methoxy, halogen, cyano, methyl, trifluoromethyl, isopropyl or nitro groups;
is $C_3$-$C_9$-heteroaryl which may be substituted by 1, 2 or 3 identical or different methoxy, halogen, cyano, methyl, trifluoromethyl, isopropyl or nitro groups; or
is $C_1$-$C_{10}$-alkyl, alkenyl or alkynyl, which may be substituted by 1, 2 or 3 identical or different methoxy, halogen, cyano, methyl, trifluoromethyl, isopropyl or nitro groups

and the compounds of the formula I may be present in the R or S form, which comprises

I) reacting mercaptans of the formula II

$$R^1\text{-SH} \qquad \text{II}$$

in which $R^1$ is as defined for the formula I in the presence of a base with a compound of the formula III

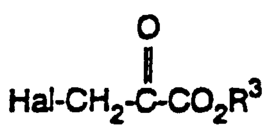

III

in which Hal may be iodine, bromine or chlorine and $R^3$ may be a straight-chain or branched alkyl radical having 1 to 6 carbon atoms, to give a compound of the formula IV

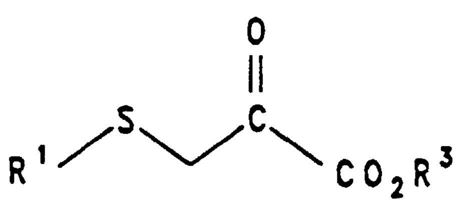

IV

in which $R^1$ is as defined for formula I and $R^3$ is as defined for formula III, and

II) reacting the compounds of the formula IV with ylides of the formula V

$$R^2\text{-CH=P}(C_6H_5)_3 \qquad V$$

in which $R^2$ is as defined for the formula I, to give the unsaturated compounds of the formula VI

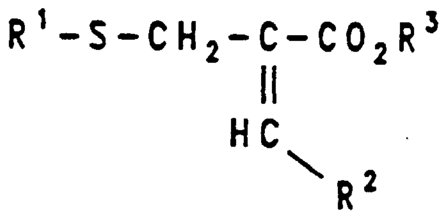

VI

in which $R^1$ and $R^2$ are as defined for formula I and $R^3$ is as defined for formula III, and

III) converting the compounds of the formula VI into compounds of the formula I, by

A) subjecting the compounds of the formula VI to alkaline hydrolysis to give compounds of the formula VII

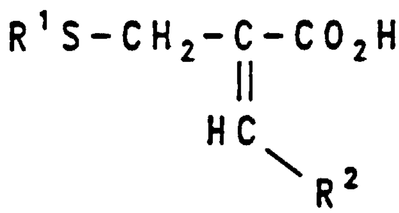

VII

in which $R^1$ and $R^2$ are as defined for the formula I, and

a) by enantioselective hydrogenation, converting compounds of the formula VII into compounds of the formula X

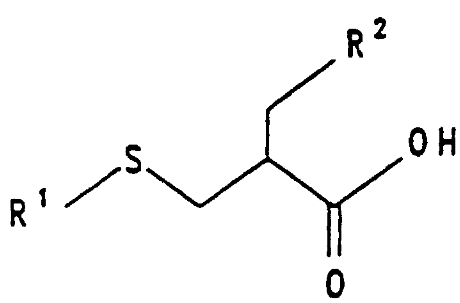

X

in which $R^1$ and $R^2$ are as defined for the formula I, and, by subsequent oxidation, converting compounds of the formula X into compounds of the formula I,
or
b) by oxidation, reacting compounds of the formula VII to give compounds of the formula XI

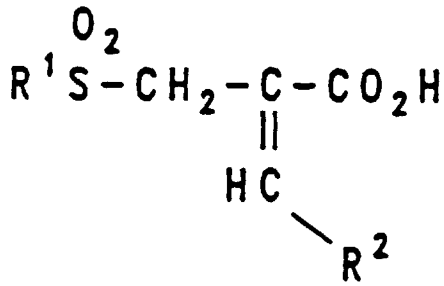

XI

in which $R^1$ and $R^2$ are as defined for the formula I, and, by subsequent enantioselective hydrogenation of the compounds of the formula XI, obtaining compounds of the formula I,
or

B) subjecting the compounds of the formula VI to enantioselective hydrogenation with chiral catalysts to give the compounds of the formula VIII

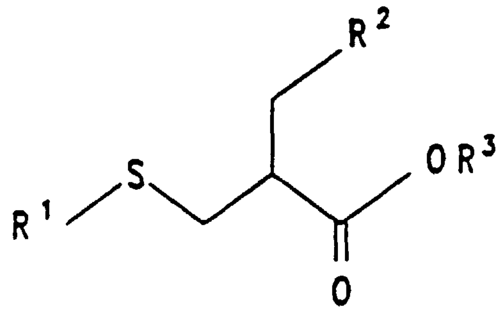

VIII

in which $R^1$ and $R^2$ are as defined for the formula I and $R^3$ is as defined for the formula III,
and

a) by alkaline hydrolysis, converting compounds of the formula VIII into compounds of the formula X

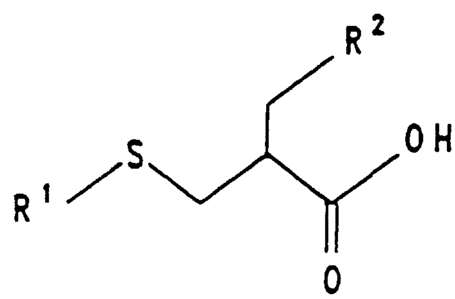

X

in which $R^1$ and $R^2$ are as defined for the formula I and, by subsequent oxidation of the compounds of the formula X, obtaining compounds of the formula I,
or
b) by oxidation of the compounds of the formula VIII, obtaining compounds of the formula XII

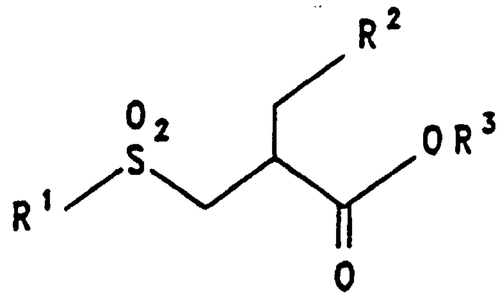

XII

in which $R^1$ and $R^2$ are as defined for the formula I and $R^3$ is as defined for the formula III, and subsequently, by alkaline hydrolysis, converting compounds of the formula XII into compounds of the formula I,
or

C) oxidizing the compounds of the formula VI to give the compounds of the formula IX

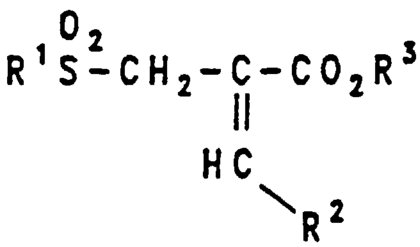

IX

in which $R^1$ and $R^2$ are as defined for the formula I and $R^3$ is as defined for the formula III,
and
converting the compounds of the formula IX into compounds of the formula I by

a) alkaline hydrolysis of the compounds of the formula IX, to give compounds of the formula XI

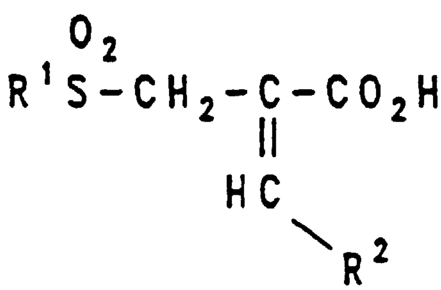

XI

in which $R^1$ and $R^2$ are as defined for the formula I, and subsequent enantioselective hydrogenation of the compounds of the formula XI to give compounds of the formula I,
or

b) enantioselective hydrogenation of the compounds of the formula IX, to give compounds of the formula XII

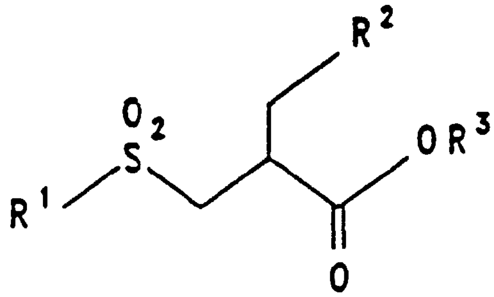

XII

in which $R^1$ and $R^2$ are as defined for the formula I and $R^3$ is as defined for the formula III, and subsequent alkaline hydrolysis of the compounds of the formula XII to give compounds of the formula I.

**2.** The process as claimed in claim 1, wherein

$R^1$ is $C_1$-$C_4$-alkyl, $C_5$-$C_6$-cycloalkyl, ($C_6$-$C_{12}$)-aryl-($C_1$-$C_3$)-alkyl or $C_6$-$C_{12}$-aryl or heteroaryl, which may be substituted by a hydroxyl, methoxy or trialkylsilyloxy group; and
$R^2$ is $C_6$-$C_{12}$-aryl which may be substituted by a methoxy, halogen, methyl, trifluoromethyl or isopropyl group; is $C_3$-$C_6$-heteroaryl which may be substituted by a methoxy, halogen, methyl, trifluoromethyl, or isopropyl group, or
is $C_1$-$C_4$-alkyl, -alkenyl or -alkynyl.

**3.** The process as claimed in claim 1 or 2, wherein

$R^1$ is $C_1$-$C_4$-alkyl,
($C_6$-$C_{12}$)-aryl-($C_1$-$C_3$)-alkyl or $C_6$-$C_{12}$-aryl, and
$R^2$ is $C_6$-$C_{12}$-aryl, $C_3$-$C_6$-heteroaryl or $C_1$-$C_4$-alkyl, -alkenyl or -alkynyl.

**4.** A process for the preparation of a compound of the formula X

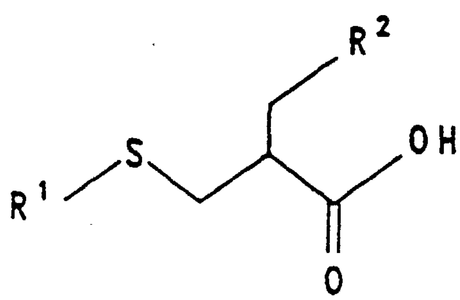

X

by enantioselective hydrogenation of a compound of the formula VII

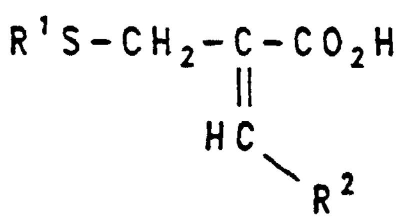

VII

in which $R^1$ and $R^2$ are as defined for formula I.

**5.** A process for the preparation of a compound of the formula VIII

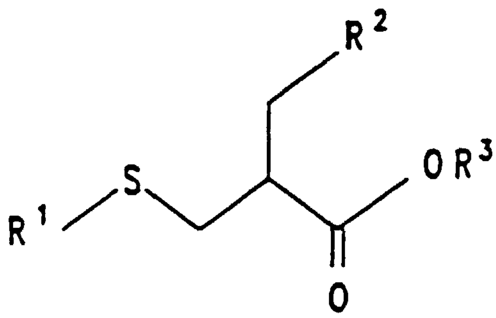

VIII

by enantioselective hydrogenation of a compound of the formula VI

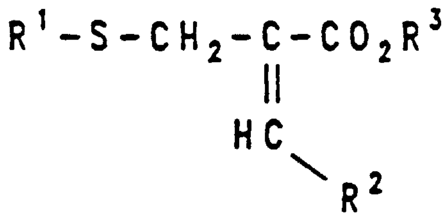

VI

in which $R^1$ and $R^2$ are as defined for formula I and $R^3$ is as defined for formula III.

**6.** A process for the preparation of a compound of the formula XII

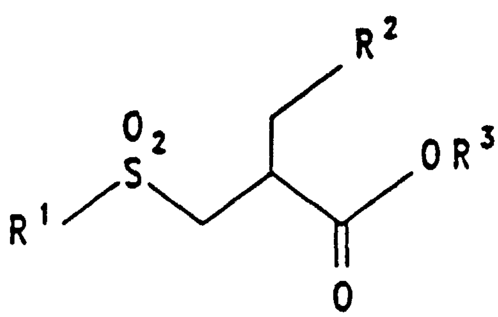

XII

by enantioselective hydrogenation of a compound of the formula IX

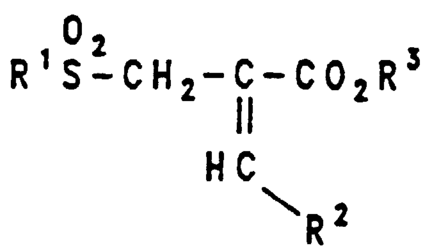

IX

in which $R^1$ and $R^2$ are as defined for formula I and $R^3$ is as defined for formula III.

**7.** A compound of the formula XI

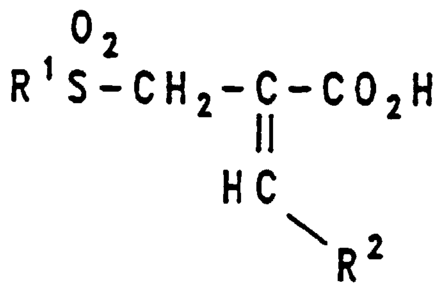

XI

in which $R^1$ and $R^2$ are as defined in claim 1.

## Revendications

**1.** Procédé pour la préparation stéréosélective d'un composé de formule I

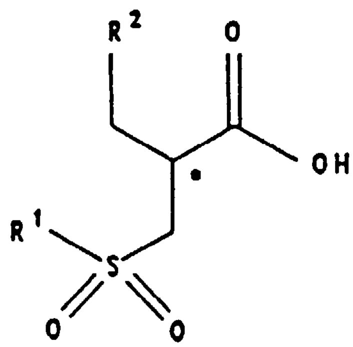

I

dans laquelle

$R^1$ représente un groupe alkyle en $C_1$-$C_6$, cycloalkyle en $C_3$-$C_6$, aryl($C_6$-$C_{12}$)alkyle($C_1$-$C_4$) ou aryle en $C_6$-$C_{12}$ ou hétéroaryle en $C_6$-$C_{12}$ ou hétérocycloalkyle en $C_6$-$C_{12}$, qui peuvent être substitués par 1, 2 ou 3 groupes hydroxyle, méthoxy ou trialkylsilyloxy, identiques ou différents; et

$R^2$ représente un groupe aryle en $C_6$-$C_{12}$ qui peut être substitué par 1, 2 ou 3 groupes méthoxy, halogéno, cyano, méthyle, trifluorométhyle, isopropyle ou nitro, identiques ou différents;

un groupe hétéroaryle en $C_3$-$C_9$ qui peut être substitué par 1, 2 ou 3 groupes méthoxy, halogéno, cyano, méthyle, trifluorométhyle, isopropyle ou nitro, identiques ou différents; ou
un groupe alkyle, alcényle ou alcynyle en $C_1$-$C_{10}$, qui peut être substitué par 1, 2 ou 3 groupes méthoxy, halogéno, cyano, méthyle, trifluorométhyle, isopropyle ou nitro, identiques ou différents,

et les composés de formule I peuvent se trouver sous la forme R ou S,
caractérisé en ce que

I) on fait réagir des mercaptans de formule II

$$R^1SH \qquad II$$

dans laquelle $R^1$ a la signification donnée à propos de la formule I, en présence d'une base, avec un composé de formule III

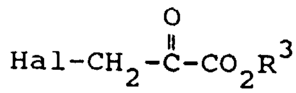

III

dans laquelle Hal peut être l'iode, le brome ou le chlore, et $R^3$ peut être un radical alkyle à chaîne droite ou ramifié, ayant de 1 à 6 atomes de carbone, pour aboutir à un composé de formule IV

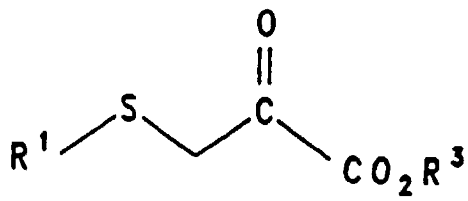

IV

dans laquelle $R^1$ a la signification donnée à propos de la formule I et $R^3$ a la signification donnée à propos de la formule III, et

II) on fait réagir les composés de formule IV avec des ylures de formule V

$$R^2\text{-CH=P}(C_6H_5)_3 \qquad \text{V}$$

dans laquelle $R^2$ a la signification donnée à propos de la formule I, pour aboutir aux composés insaturés de formule VI

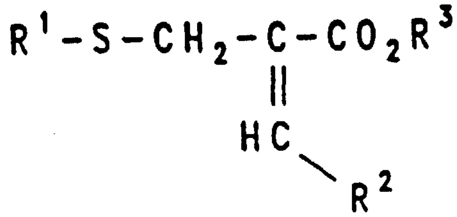

VI

dans laquelle $R^1$ et $R^2$ ont les significations données à propos de la formule I et $R^3$ a la signification donnée à propos de la formule III, et

III) on convertit les composés de formule VI en composés de formule I

A) en soumettant à une saponification alcaline les composés de formule VI, pour aboutir aux composés de formule VII,

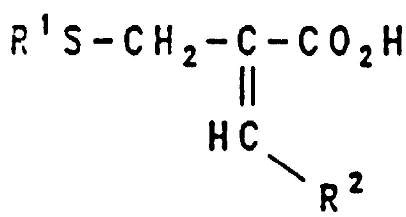

VII

dans laquelle $R^1$ et $R^2$ ont les significations données à propos de la formule I, et

a) en convertissant par hydrogénation énantiosélective les composés de formule VII en composés de formule X

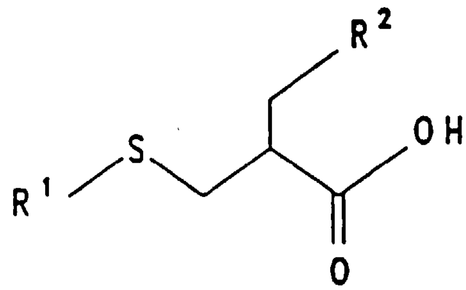

X

dans laquelle $R^1$ et $R^2$ ont les significations données à propos de la formule I, et en convertissant par oxydation subséquente les composés de formule X en composés de formule I,
ou
b) en convertissant par oxydation les composés de formule VII en composés de formule XI

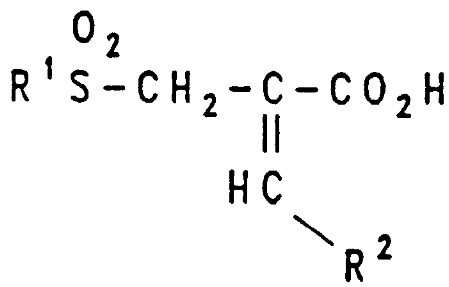

XI

dans laquelle $R^1$ et $R^2$ ont les significations données à propos de la formule I, et, par hydrogénation énantiosélective subséquente des composés de formule XI, on obtient des composés de formule I,
ou

B) en soumettant à une hydrogénation énantiosélective les composés de formule VI, avec des catalyseurs chiraux, pour aboutir aux composés de formule VIII

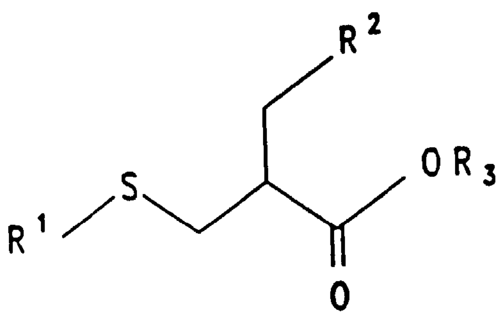

VIII

dans laquelle $R^1$ et $R^2$ ont les significations données à propos de la formule I et $R^3$ a la signification donnée à propos de la formule III,
et

a) en convertissant par saponification alcaline les composés de formule VIII en composés de formule X

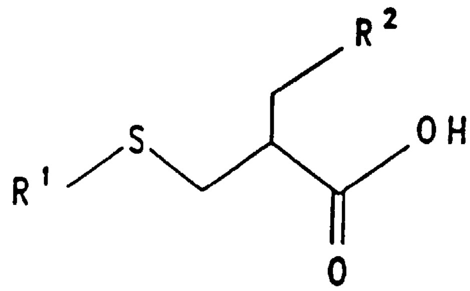

X

dans laquelle $R^1$ et $R^2$ ont les significations données à propos de la formule I, et, par oxydation subséquente des composés de formule X, on obtient des composés de formule I,
ou
b) par oxydation des composés de formule VIII, on obtient des composés de formule XII,

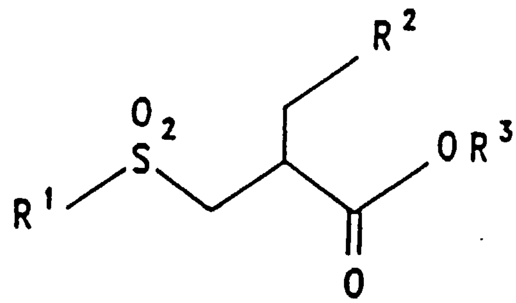

XII

dans laquelle $R^1$ et $R^2$ ont les significations données à propos de la formule I et $R^3$ a la signification donnée à propos de la formule III, et
ensuite, par saponification alcaline, on convertit les composés de formule XII en composés de formule I,
ou

C) en oxydant les composés de formule VI en les composés de formule IX,

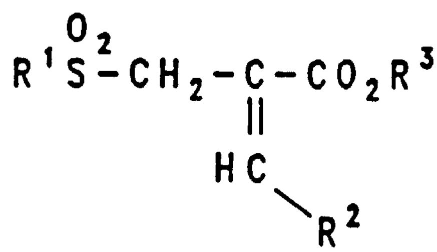

IX

dans laquelle $R^1$ et $R^2$ ont les significations données à propos de la formule I et $R^3$ a la signification donnée à propos de la formule III,
et
en convertissant les composés de formule IX en composés de formule I par

a) saponification alcaline des composés de formule IX conduisant aux composés de formule XI,

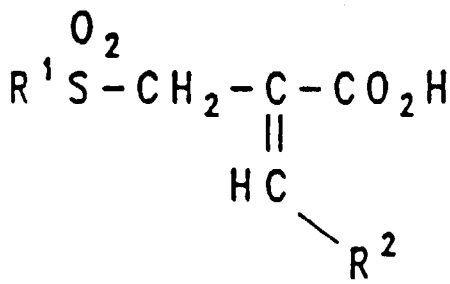

XI

dans laquelle $R^1$ et $R^2$ ont les significations données à propos de la formule I, et hydrogénation énantiosélective subséquente des composés de formule XI, conduisant aux composés de formule I,
ou
b) hydrogénation énantiosélective des composés de formule IX conduisant aux composés de formule XII,

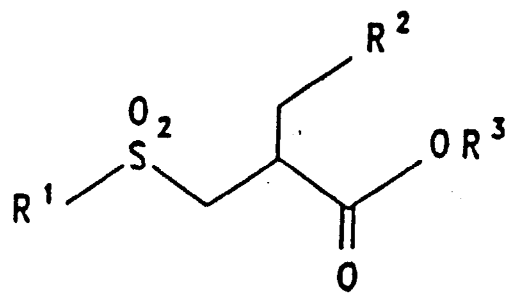

XII

dans laquelle $R^1$ et $R^2$ ont les significations données à propos de la formule I et $R^3$ a la signification donnée à propos de la formule III, et saponification alcaline subséquente des composés de formule XII, conduisant aux composés de formule I.

**2.** Procédé selon la revendication 1, caractérisé en ce que

$R^1$ représente un groupe alkyle en $C_1$-$C_4$, cycloalkyle en $C_5$-$C_6$, aryl($C_6$-$C_{12}$)alkyle($C_1$-$C_3$) ou aryle en $C_6$-$C_{12}$ ou hétéroaryle en $C_6$-$C_{12}$, qui peuvent être substitués par un groupe hydroxyle, méthoxy ou trialkylsilyloxy;
$R^2$ représente un groupe alkyle en $C_6$-$C_{12}$ qui peut être substitué par un atome d'halogène ou par le groupe méthoxy, méthyle, trifluorométhyle ou isopropyle; un groupe hétéroaryle en $C_3$-$C_6$ qui peut être substitué par un atome d'halogène ou par le groupe méthoxy, méthyle, trifluorométhyle ou isopropyle, ou un groupe alkyle, alcényle ou alcynyle en $C_1$-$C_4$.

**3.** Procédé selon la revendication 1 ou 2, caractérisé en ce que

$R^1$ représente un groupe alkyle en $C_1$-$C_4$, aryl($C_6$-$C_{12}$)alkyle($C_1$-$C_3$) ou aryle en $C_6$-$C_{12}$, et
$R^2$ représente un groupe aryle en $C_6$-$C_{12}$, hétéroaryle en $C_3$-$C_6$ ou alkyle, alcényle ou alcynyle en $C_1$-$C_4$.

**4.** Procédé pour la préparation des composés de formule X

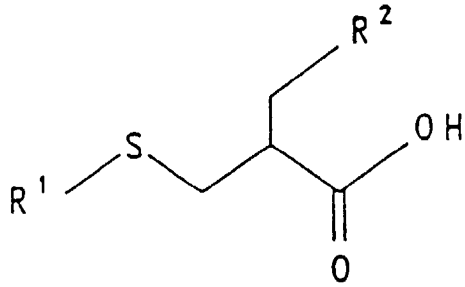

X

par hydrogénation énantiosélective d'un composé de formule VII

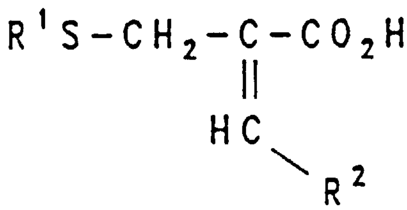

VII

dans laquelle $R^1$ et $R^2$ ont les significations données à propos de la formule I.

**5.** Procédé pour la préparation des composés de formule VIII

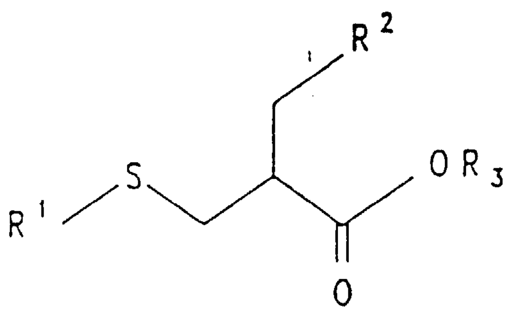

VIII

par hydrogénation énantiosélective d'un composé de formule VI

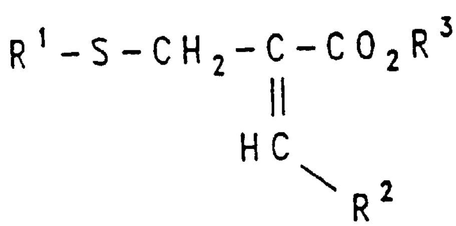

VI

dans laquelle $R^1$ et $R^2$ ont les significations données à propos de la formule I, et $R^3$ a les significations données à propos de la formule III.

**6.** Procédé pour la préparation des composés de formule XII

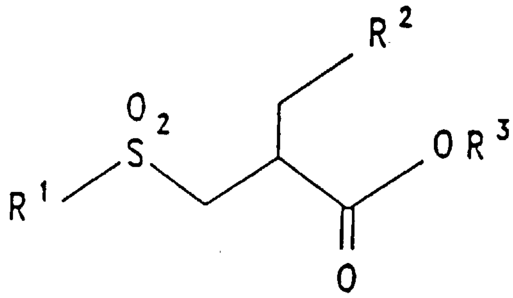

XII

par hydrogénation énantiosélective d'un composé de formule IX

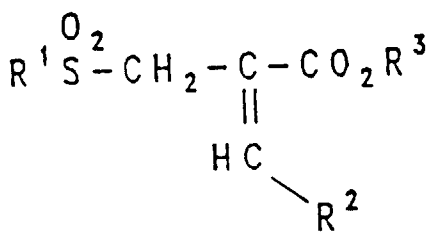

IX

dans laquelle $R^1$ et $R^2$ ont les significations données à propos de la formule I, et $R^3$ a les significations données à propos de la formule III.

**7.** Composés de formule XI

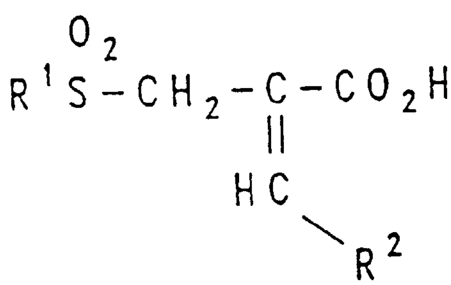

XI

dans laquelle $R^1$ et $R^2$ ont les significations données dans la revendication 1.